(19) Europäisches Patentamt
European Patent Office
Office européen des brevets

(11) EP 1 441 036 A1

(12) # EUROPEAN PATENT APPLICATION

(43) Date of publication:
**28.07.2004 Bulletin 2004/31**

(51) Int Cl.[7]: **C12Q 1/68**, G06F 19/00

(21) Application number: **03290203.3**

(22) Date of filing: **27.01.2003**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(71) Applicant: **Université de Nantes**
**44035 Nantes (FR)**

(72) Inventors:
- **Sakanyan, Vehary**
  **44700 Orvault (FR)**
- **Dekhtyar, Mikael**
  **Tver - 170032 (RU)**
- **Morin, Amélie**
  **44000 Nantes (FR)**
- **Braun, Frédérique**
  **44000 Nantes (FR)**
- **Modina, Larissa**
  **44300 Nantes (FR)**

(74) Representative: **Desaix, Anne et al**
**Ernest Gutmann - Yves Plasseraud S.A.**
**3, rue Chauveau-Lagarde**
**75008 Paris (FR)**

(54) **Method for the identification and isolation of strong bacterial promoters**

(57) The present invention relates to the identification and the isolation from bacterial genomes of new sequences having strong bacterial promoter activity. The invention also concerns new nucleic acids having strong bacterial promoter activity and their uses for improving RNA and/or protein synthesis using cellular (*in vivo*) or cell-free (*in vitro*) expression systems.

EP 1 441 036 A1

## Description

The present invention relates to the identification and the isolation from bacterial genomes of new sequences having strong bacterial promoter activity. The invention also concerns new nucleic acids having strong bacterial promoter activity and their uses for improving RNA and/or protein synthesis using cellular (*in vivo)* or cell-free *(in vitro)* expression systems.

Recombinant protein production in bacterial cells is a major area of biotechnology. Examples of recombinant molecules of interests synthesized in bacteria are antigens, antibodies and fragments thereof for vaccines, enzymes in medicine or agro-food industry, hormones, cytokines or growth factor in medicine or agronomy.

High throughput technologies and in particular protein array methods for analyzing protein-molecules interactions (EP 01402050.7), needs also to provide protein or polypeptide of interest, such as an antigen, an antibody, a receptor for identifying ligands, agonists or antagonists thereof.

Synthesis of a desired mRNA can also be convenient for their subsequent use in protein synthesis, in diagnosis or in anti-sense therapeutic approach for example.

Many microbial overexpression systems have been developed to achieve high yield of protein synthesis.

Usual methods of recombinant protein synthesis include *in vivo* expression of recombinant genes from strong promoters in corresponding host cells, such as bacteria, yeast or mammalian cells or *in vitro* expression from a DNA template in cell-free extracts, such as the S30 system-based method developed by Zubay (1973), the rabbit reticulocyte system-based method (Pelham and Jackson, 1976) or wheat germ lysate system-based method (Roberts and Paterson, 1973). Cell-free synthesis has been applied for polysome display screening antibodies (Mattheakis *et al*., 1996), truncation test (van Essen *et al*., 1997), scanning saturation mutagenesis (Chen *et al*., 1999), site-specific incorporation of unnatural amino acids into proteins (Thorson *et al*., 1998), stable-isotope labeling of proteins (Kigawa *et al*., 1999) and protein array screening molecular interactions (EP 01402050.7).

The best known expression systems in the art are based on the use of strong transcriptional signals. As an example, strong phage promoters are widely used for gene expression and protein production both in living cells or cell-free extracts.

However, improvements at the different steps of gene expression are still required to increase the yield of RNA or protein synthesis in an expression system as well as to improve the performance of overexpression of a given protein. If the different components involved in transcription are well-known in the Art, the specific contribution of each component is still controversial.

Transcription initiation can be considered as one of the rate-limiting step in mRNA synthesis, thereby for protein synthesis as well. Therefore, identification and use of strong promoters in microbial genomes can lead to the development of new *in vivo* and *in vitro* protein overexpression systems. Furthermore, studying strong promoters is important for the elucidation of a global transcriptional regulation of highly expressed genes and operons in the context of a whole organism and further improving the performance of protein overexpression in cellular as well as in cell-free systems.

RNA polymerase is a unique enzyme required for transcription of genes in all bacteria. Its core-enzyme consists of subunits $\alpha$ (in a dimeric state), $\beta'$, $\beta$ and $\omega$, which binds exchangeable $\sigma$ subunits and forms a holoenzyme able to recognize a promoter sequence and to initiate transcription. The assemblage of a core enzyme occurs in the following order $\alpha$ $\alpha 2$ $\alpha 2\beta$ $\alpha 2\beta\beta'$ (Kimura et Ishihama, 1996). In a majority of promoters, consensus sequences TATAAT (site -10) et TTGACA (site -35) determine the recognition of a major $\sigma$ subunit considered as an analogue of *Escherichia coli* $\sigma^{70}$ factor.

The strength of a major $\sigma$-dependent bacterial promoters is determined by a rate of homology of their -10 et -35 sites with corresponding consensus sequences and by the length of a distance (spacer) between these sites that should be 17 ± 1 bp. However, the strong promoter recognition depends also on binding RNA polymerase $\alpha$ subunit to a 17-20 bp AT-rich sequence located just upstream the -35 site and known as a UP-element (Ross *et al*., 1993). A consensus sequence 5'NNAAAWWTWTTTTNNNAAANNN (where W is A or T and N is any of four bases) was established for *E. coli* UP element by sequence analysis of artificially created sequences providing high gene expression (Estrem *et al*., 1998). This consensus can be divided into two parts, a proximal AAAAAARNR (where R is A or G) and a distal subsite NNAWWWWWTTTTTN (Estrem *et al*., 1999). Searching for similar sequences located upstream of previously detected promoters in the *E. coli* genome (Thieffry *et al.,* 1998; *http://www.cifn.unam.mx/Computational Biology/E.coli-predictions)* with a software GCG version 9.0 allowed to detect 32 putative promoters having 4 mismatches in the full UP element consensus (Estrem *et* al., 1999). Extended AT-rich sequences, which can be considered as UP elements or UP element-like sequences have been also detected in bacteria *Clostridium pasterianum* (Graves *et al*., 1986), *Bacillus subtilis* (Fredrick *et al*., 1995), *Bacillus stearothermophilus* (Savchenko *et al*., 1998) and *Vibrio natrigens* ( Aiyar *et al*., 2002). The presence of such a sequence in a promoter can rise up to 330-fold gene expression in *Escherichia coli* cells (Aiyar *et al*., 1998). The N-terminal domain of $\alpha$ subunit is responsible for assemblage of RNA polymerase whereas the C-terminal domain is implicated into contacts to UP-element and other transription activators (Ross *et al.,* 2001).

Thus, a UP-element of strong promoters seems to play an essential role in the modulation of the level of mRNA synthesis in bacterial cells.

Consequently, in the present invention, it has been further confirmed that the α subunit of RNA polymerase plays a determinant role in increasing RNA and protein synthesis in cell-free systems, as compared to the other subunits of a core-enzyme of RNA polymerase.

As used herein, a "cellular system for *in vivo* RNA or protein synthesis" refers to a system enabling RNA or protein synthesis including a host cell comprising an appropriate recombinant DNA template for the expression of a gene of interest and subsequent synthesis of RNA or protein of interest.

As used herein, a "cell-free system" or "cell-free synthesis system" refers to any system enabling the synthesis of a desired protein or of a desired RNA from a DNA template using cell-free extracts, namely cellular extracts which do not contain viable cells. Hence, it can refer either to *in vitro* transcription-translation or *in vitro* translation systems. Examples of eucaryotic *in vitro* translation methods are based on the extracts obtained from rabbit reticulocytes (Pelham and Jackson, 1976), or from wheat germ cells (Roberts and Paterson, 1973). The *E. coli* S30 extract-based method described by Zubay (1973) is an example of a widely used prokaryotic *in vitro* translation method.

The term "protein" refers to any amino-acid sequence.

The inventors have now developed new tools for the identification of nucleic acid sequences carrying putative strong bacterial promoter. The inventors have also isolated nucleic acid sequences having strong bacterial promoter activity.

As used herein the term "nucleic acid" or "nucleic acid sequence" includes RNA, DNA fragment, polynucleotide or oligonucleotide, cDNA, genomic DNA and messenger RNA.

For suitable reading of the present text, the chemical structure of a nucleic acid will be characterized by a nucleotide sequence represented by a chain of "A", "G", "C" or "T", as usual for the one skilled in the Art. Of course, when a sequence is given for a double-strand DNA, it implicitely means that the reverse complementary sequence forms the other strand of such DNA.

The term "promoter" or "promoter activity" is used in the present text to refer to the capacity of a nucleic acid when inserted immediately upstream an Open Reading Frame or a sequence coding for tRNA or rRNA to promote transcription of said sequences.

Method for measuring promoter activity are well-known in the Art. The promoter activity can be measured for example according to the method below:

- The nucleic acid whose promoter activity is measured, is placed immediately upstream an Open Reading Frame of a reporter gene,
- The resulting construction is placed in an appropriate vector and introduced into *E. coli* cells,
- The *E. coli* cells are cultured in conditions appropriate for expression of the reporter gene,
- Transcriptional expression of the reporter gene is determined and compared with the transcriptional expression of the same reporter gene placed downstream a control promoter.

Instead of determining transcriptional expression, it is also possible to determine protein synthesis of a reporter protein, since transcriptional activation is usually the rate limiting step for protein synthesis. A specific method for measuring the promoter activity of a nucleic acid in a cell-free system by determining protein synthesis of ArgC reporter protein is described in the example.

According to the present invention, a nucleic acid is considered to have a strong bacterial promoter activity when transcriptional expression of a gene inserted downstream said nucleic acid is higher than the transcriptional expression of the same gene inserted downstream a control bacterial strong promoter, such as the ptac promoter.

A first object of the invention is a method for the identification of a nucleic acid sequence carrying a putative bacterial strong promoter, said method comprising:

a. selecting among the sequences of a nucleic acid database, a putative promoter sequence of at least 50 nucleotides, preferably around 60-70 nucleotides, said putative promoter sequence being located upstream the initiation codon of an Open Reading Frame or a sequence corresponding to tRNA or rRNA, in a region which does not extend further than 500 nucleotides, preferably 300 nucleotides from said initiation codon, said putative promoter sequence comprising an UP element, said UP element consisting of either

- the following consensus pattern: AAAWWTWTTTTNNNAAA (SEQ ID NO:1), wherein "W" stands for any of the symbols "A" or "T" and "N" stands for any of the four symbols "A", "T", "G" or "C"; or,

- a nucleotide sequence of the same length of SEQ ID NO:1 which can be aligned with SEQ ID NO:1 and having a score similarity *sUP* which is equal or superior to a minimal score similarity parameter *scUP*,

b. selecting among the sequences selected in step a., the sequences comprising a -35 site located from 0 to 5 nucleotides downstream the UP element, said -35 site consisting of either

- the following consensus pattern TCTTGACAT (SEQ ID NO:2), or
- a nucleotide sequence of the same length of SEQ ID NO:2 which can be aligned with SEQ ID NO:2 and having a score similarity s35 which is equal or superior to a minimal score similarity parameter sc35; and

c. identifying among the sequences selected in step b., a sequence comprising a -10 site, downstream the -35 site, preferably at a distance from 14 to 20 nucleotides, preferably from 15 to 19, better from 16 to 18, and optimally 17 nucleotides from the -35 site, said - 10 site consisting of either

- the following consensus pattern TATAAT (SEQ ID NO:3), or
- a nucleotide sequence of the same length of SEQ ID NO:3 which can be aligned with SEQ ID NO:3 and having a score similarity *s10* which is equal or superior to a minimal score similarity parameter *sc10*.

As used herein, the term "putative strong promoter" means that there is a high probability the sequence carry a strong promoter.
As used herein, the term "nucleic acid database" means a database which gathers sequence information obtained by the sequencing of nucleic acids. Especially, the database gathers genomic sequences information. Databases from micro-organism genomes such as prokaryotes are especially preferred.
In a preferred embodiment, searched nucleic acid databases are selected among the genome having a percentage of adenine and thymine inferior to 65%, more preferably, inferior to 50%. Indeed, it has been shown that these databases enable the identification of a high number of strong promoters.
Preferably, the nucleic acid databases comprise genomic sequences from bacterial species from bacteria which is used in the industry and whose genome comprises a percentage of adenine and thymine inferior to 65%.
Examples of such bacterias are listed in Table 5.
Particularly are preferred bacterial nucleic acid databases comprising genomic sequences from one bacterial specie selected from the group consisting of *Thermatoga maritima, Mycobacterium tuberculosis, Mycobacterium leprae, Pseudomonas aeruginosa, Brucella melitensis, Neisseria meningitis, Salmonella typhimurium, Escherichia coli, Vibrio cholerae, Yersinia pestis, Streptococcus pneumoniae, Streptococcus pyogenes, Haemophilus influenzae* and *Helicobacter pylori*.
One example of the present invention is the use of the method for identifying nucleic acid sequence from bacterial nucleic acid database of *T. maritima* genomic sequences.
The similarity scores between two aligned sequences referred by *sUP, s35* and *s10* correspond to the sum of each coincidence rates of symbols in the corresponding alignments: the identity rate is equal to 1, the non-identity rate is 0.5 or 0 and is determined for each pair of compared symbols as follows:

0.5 for pairs "A" to "T" or
"T" to "A" and
0 for other possible pairs.

Therefore, the similarity score between each consensus pattern and the aligned sequence varies from 0 to the corresponding length of the pattern, namely 17 for UP element, 9 for -35 site and 6 for -10 site.
The minimal acceptable value for *sUP, s35* and *s10* for selecting the putative promoter are defined by the parameters *scUP, sc35* and *sc10* which can be determined empirically depending upon the nature of the database, the size of the database, the number and the strength of promoters to be identified by the method.
In a preferred embodiment of the method, *scUP* is at least equal to 11, *sc35* is at least equal to 5, and *sc10* is at least equal to 4. Such combination of parameters for minimal score similarity are particularly preferred for the screening of databases of *Thermotoga maritima* genomic sequences.
In a particular embodiment of the method, a normalised score is attributed to each identified sequence enabling the comparison of the putative strength for each identified sequence.
According to one specific embodiment of the method, a normalised score tot_sc is attributed to each identified sequence according to the following equation:

$$tot_sc = 0.30*[1-(17- sUP)/20] + 0.25*[1-(9- s35)/10] + 0.25*[1-(6- s10)^2/10]$$

+ 0.2*nsc_dist,

wherein nsc_dist is defined according to the following table 1 :

| Distance between -35 site and -10 site in nucleotides | 17 | 16, 18 | 15, 19 | 14, 20 | Other |
|---|---|---|---|---|---|
| *nsc_dist* | 1 | 0.95 | 0.85 | 0.7 | 0.2 |

and the method further comprises the step of selecting the sequences having a normalised score tot_sc superior to 0.85.

Of course, any other methods of calculation of the normalised score which enable similar comparison of the strength of the identified promoters can be applied.

The formula of the normalized score should reflect the inexact matching for the different subregions, e.g., the UP element, the -35 site and the -10 site and the relative importance of corresponding subregions and the spacer for the evaluation of the promoter strength. The rate of similarity for each subregion can be modulated by increasing or decreasing the attached coefficients. However, it has been shown that the set of sequences having strong promoter activity identified by the method of the invention does not essentially depend upon small variation of the coefficients.

Indeed, the inventors have shown that a majority of promoters identified from *T. maritima* genome and having a score superior to 0.85 according to the above-defined equation, have strong promoter activity.

Naturally, the invention also relates to a computer program comprising computer program code means for instructing a computer to perform the method of the invention.

The invention further concerns a computer readable storage medium having stored therein a computer program according to the invention.

Another aspect of the invention is a method for the isolation of a nucleic acid having strong bacterial promoter activity, wherein said method further comprises the steps of:

a. isolating a nucleic acid having a putative strong bacterial promoter, said nucleic acid sequence being identified according to the method defined above,
b. determining promoter activity of the isolated nucleic acid as compared to a control bacterial strong promoter, such as the ptac promoter,

wherein a higher promoter activity than the promoter activity of the control strong promoter indicates that said isolated nucleic acid has a strong bacterial promoter activity.

Any appropriate means for determining promoter activity of said isolated nucleic acid can be used for the method of the invention. A preferred method is described in an example as the detection of synthesis of the reporter protein ArgC in a cell-free system. Obviously, other reporter protein can also be used.

By implementing the method of the invention, the inventors have identified new nucleic acids derived from *T. maritima* genomic sequence, having a strong bacterial promoter activity.

Another aspect of the invention thus relates to an isolated nucleic acid having a strong bacterial promoter activity, characterized in that it is obtainable by the method defined above and in that it consists of

a. a nucleic acid sequence selected among the group consisting of SEQ ID NOs 4-16;
b. a modified nucleic acid sequence having at least 70%, preferably at least 80%, and better at least 90% identity when aligned with one of SEQ ID NOs 4-16,
c. a modified nucleic acid sequence which hybridizes under stringent conditions with one of the sequences of SEQ ID NOs 4-16, or,
d. a nucleic acid sequence comprising the following consensus pattern: GNAAAAAtWTNTTNAAAAAAMNCTT-GAMA$(N)_{18}$TATAAT (SEQ ID NO:21) wherein "W" stands for any of the symbols "A" or "T", "N" stands for any of the four symbols "A", "T", "G" or "C" and "M" stands for "A" or "C", wherein said modified nucleic acid is between 50 and 300 nucleotides long, preferably between 50 and 100 nucleotides long, and retains substantially the same promoter activity as the non-modified sequence to which it can be aligned.

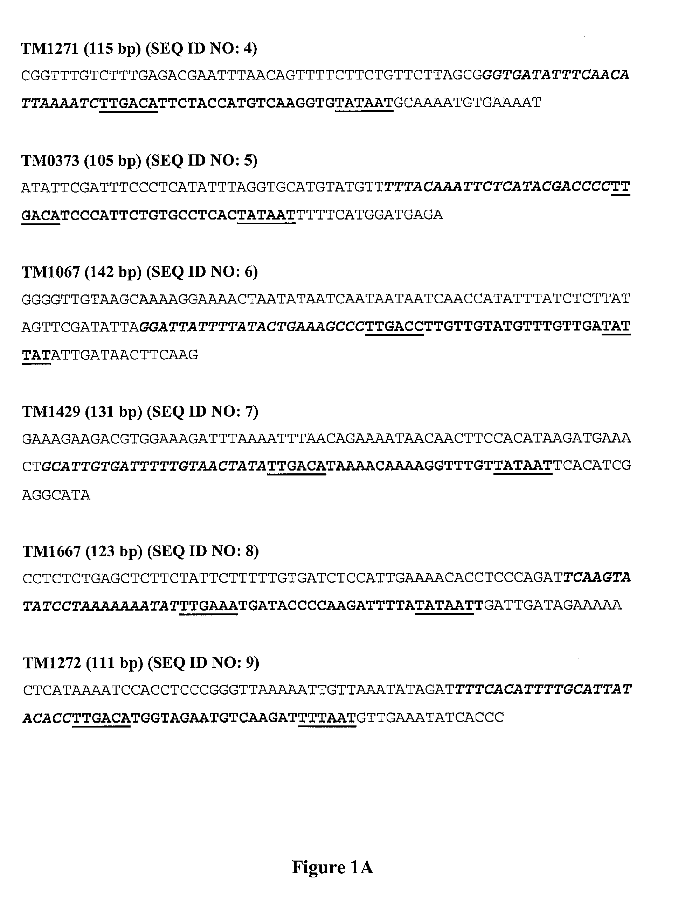

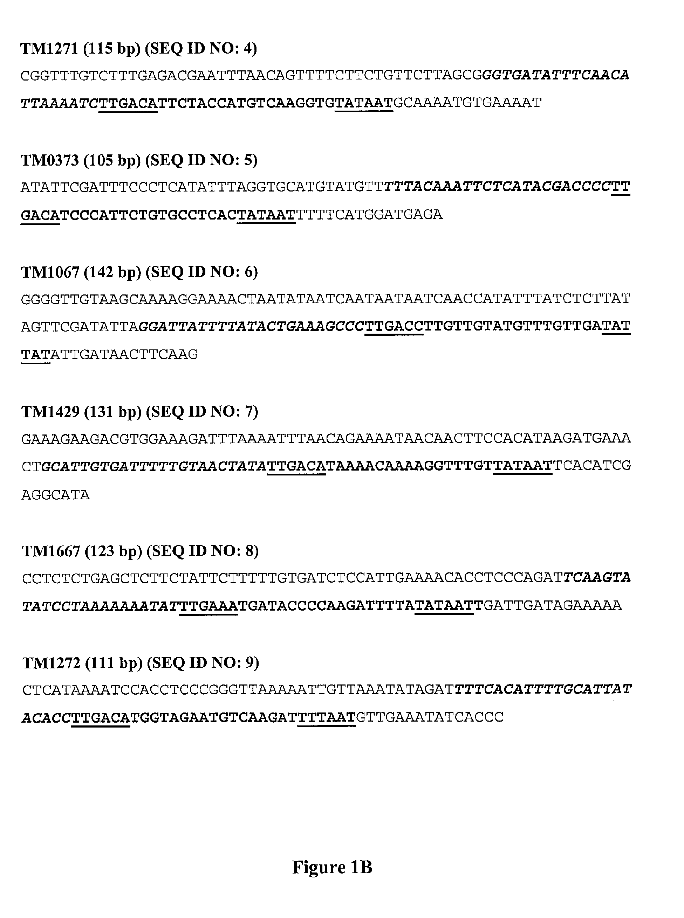

The nucleic acid of SEQ ID NOs 4-16 are more specifically defined in figure 1 and in example 2.

For evaluating the similarity of a modified sequence with one of SEQ ID Nos 4-16, the alignment program BLASTA (Altschul et al., 1990) is used.

As used herein, the term « stringent conditions » refers to the conditions enabling specific hybridisation of the single strand nucleic acid at 65°C for example in a solution consisting of 6X SSC, 0.5% SDS, 5X Denhardt's solution and 100 mg of non specific DNA carrier, or any other solution of the same ionic strength, and after a washing at 65°C, for example in a solution consisting of 0.2X SSC and 0.1 SDS or any other solution of the same ionic strength. The parameters which define the stringency conditions are the temperature at which 50% of the stands are separated (Tm). For nucleic acids more than 30 bases, Tm is defined as follows: Tm = 81,5 + 0,41 (%G+C) + 16,6Log (concentration in cations) - 0.63(% formamide) - (600/number of bases). Stringency conditions can be adapted according to the size of the sequence and the content of GC and all other parameters, according to the protocols described in Sambrook *et al*.

Modified nucleic acids derived from SEQ ID NOs 4-16 which retains substantially the same promoter activity as the non-modified from which it can be aligned are also concerned by the present invention.

According to the invention, it will be considered that a modified sequence retains substantially the same promoter activity as the non modified sequence from which it can be aligned if the measured promoter activity is not inferior to 70%, preferably 80%, and more preferably 90% than that of the non-modified sequence to which it can be aligned.

Of course, modified sequence having a higher promoter activity than the non-modified sequence from which it can be aligned are comprised in the present invention.

Preferably, such modified sequence is a sequence which has been modified by deletion or mutagenesis. Preferred modifications are nucleotides substitutions which do not fall in the regions comprising the UP element, the -35 site and the - 10 site as defined above. Other preferred modifications are nucleotide substitutions which increase similarity of the UP element, -35 site or the - 10 site with the corresponding consensus pattern as defined above. Another preferred modification is a modification of the length of the distance separating the -35 and the -10 site to render it closer to the optimal distance of 17 $\pm$1 nucleotides.

Naturally, such preferred modifications would not necessarily increase the strength of the promoter, but the one skilled in the Art can screen the promoter activity of the modified sequence, in order to select the appropriate modifications.

The nucleic acid having strong bacterial promoter activity are more specifically useful for the synthesis of a protein and/or RNA of interest.

Another aspect of the invention is thus an expression cassette comprising a nucleic acid having strong bacterial promoter activity according to the invention.

As used herein, an expression cassette is a means for inserting into, a sequence encoding a protein of interest and for synthesizing said protein into a host cell or in a cell-free system.

The expression cassette preferably is a DNA molecule containing a multiple cloning site immediately downstream the nucleic acid having strong bacterial promoter activity of the invention. The multiple cloning site enables the insertion using restriction enzymes and ligase of the sequence encoding the protein of interest.

Preferably, the expression cassette is characterized in that it is a plasmid, a cosmid or a phagemid for *in vivo* protein synthesis.

Advantageously, the expression cassette of the invention further comprises an Open Reading Frame encoding $\alpha$ subunit of a RNA polymerase under the control of a promoter appropriate for expression in said host cell.

The invention also relates to a DNA template for RNA or protein synthesis, comprising the nucleic acid having strong bacterial promoter activity of the invention, inserted upstream an Open Reading Frame encoding a protein of interest.

According to the invention, a "protein of interest" refers to any type of protein characterised in that it is not naturally expressed from the nucleic acid having strong bacterial promoter activity of the invention.

Examples of protein of interest are enzymes, enzyme regulators, receptor ligands, haptens, antigens, antibodies and fragments thereof.

In order to simplify the reading of the present text, as used herein, the term "DNA template" refers to a nucleic acid comprising the following elements:

- an Open Reading Frame with an initiation codon and a stop codon encoding a protein of interest;
- the nucleic acid having strong bacterial promoter activity as here-aboved defined, located upstream the Open Reading Frame encoding a protein of interest;
- optionally specific signals for translation initiation and termination;
- optionally, specific signals for transcription termination ;
- optionally, specific signals for binding transcriptional activating proteins;
- optionally, a sequence in frame with said Open Reading Frame, encoding a tag for convenient purification or detection.

The selection of the different above-mentioned elements depends upon the selected expression system.

Preferably, the nucleic acid having strong bacterial promoter activity of the invention is located immediately upstream the initiation codon of the Open Reading Frame encoding the protein of interest.

In cell-free systems, linear DNA templates may affect the yield of RNA or protein synthesis and their homogeneity because of nuclease activity in the cell-free extract. By "protein homogeneity", it is meant that a major fraction of the synthesized product correspond to the complete translation of the Open Reading Frame, leading to full-length protein synthesis and only a minor fraction of the synthesized proteins correspond to interrupted translation of the Open Reading Frame, leading to truncated forms of the protein. Thus, the desired protein synthesis is less accompanied by truncated polypeptides.

The use of elongated DNA template according to the invention, improves the yield and the homogeneity of synthesized proteins in cell-free systems.

Thus, in a preferred embodiment, a linear DNA template further comprises an additional DNA fragment, which is at least 3 bp long, preferably longer than 100 bp, and more preferably longer than 200bp, located immediately downstream the stop codon of the Open Reading Frame encoding the desired RNA or protein of interest.

It has also been shown that the use of DNA template further comprising an additional DNA fragment containing transcriptional terminators, improves the yield and the homogeneity of the protein synthesis from cell-free systems.

One example of transcription terminators which can be used in the present invention is the T7 phage transcriptional terminator.

The DNA template of the invention are useful in a method for RNA or protein synthesis from a DNA template comprising the steps of

a. providing a cellular or cell-free system enabling RNA or protein synthesis from the DNA template according to the invention;

b. recovering said synthesized RNA or protein.

The strong bacterial promoter contained in the used DNA template are particularly efficient to bind α subunit of RNA polymerase.

In a preferred embodiment, in order to increase the yield of RNA and/or protein synthesis, the concentration of α subunit of RNA polymerase, but not of other subunits, is increased in said cellular or cell-free system, comparing to its natural concentration.

As used herein, the term "natural concentration" refers to the concentration of the RNA polymerase α subunit established *in vivo* in bacterial cells without affecting the growth conditions or the concentration of the RNA polymerase α subunit *in vivo* reconstituted holoenzyme from purified subunits.

The increase of the concentration of the α subunit can refer, either to an increase of the concentration of an α subunit which is identical to the one initially present in the selected expression system, or to an α subunit which is different but which can associate with β, β' and ω subunits initially present in the expression system to form the holoenzyme. For example, said different α subunit can be a mutated form of the α subunit, initially present in the selected expression system or a similar form from a related organism, provided that the essential αCTD and/or αNTD domains are still conserved or a chimaeric from related organisms.

The α subunit used is, for example, obtained from *E. coli* or *T. maritima*.

Preferably, the α subunit is derived from the same organism as the one from which is derived the strong promoter used in the DNA template and which can be obtained by the method of the invention.

In one specific embodiment, said system enabling RNA or protein synthesis from the DNA template of the invention is a cellular system.

The DNA templates can be adapted for any cellular system known in the Art. The one skilled in the Art will select the cellular system depending upon the type of RNA or protein to synthesize.

In one aspect of the invention, a cellular system comprises the culture of prokaryotic host cells. Preferred prokaryotic host cells include *Streptococci, Staphylococci, Streptomyces* and more preferably, *B. subtilis* or *E. coli* cells.

In a preferred embodiment, a host cell selected for the cellular expression system is a bacteria, preferably an *Escherichia coli* cell.

Host cells may be genetically modified for optimising recombinant RNA or protein synthesis. Genetic modifications that have been shown to be useful for *in vivo* expression of RNA or protein are those that eliminate endonuclease activity, and/or that eliminate protease activity, and/or that optimise the codon bias with respect to the amino acid sequence to synthesize, and/or that improve the solubility of proteins, or that prevent misfolding of proteins. These genetic modifications can be mutations or insertions of recombinant DNA in the chromosomal DNA or extra-chromosomal recombinant DNA. For example, said genetically modified host cells may have additional genes, which encode specific transcription factors interacting with the promoter of the gene encoding the RNA or protein to synthesize.

Prior to introduction into a host cell, the DNA template is incorporated into a vector appropriate for introduction and replication in the host cell. Such vectors include, among others, chromosomic vectors or episomal vectors or virus-derived vectors, especially, vectors derived from bacterial plasmids, phages, transposons, yeast plasmids and yeast chromosomes, viruses such as baculoviruses, papoviruses and SV40, adenoviruses, retroviruses and vectors derived from combinations thereof, in particular phagemids and cosmids.

For enabling secretion of translated proteins in the periplasmic space of gram bacteria or in the extracellular environment of cells, the vector may further comprise sequences encoding secretion signal appropriate for the expressed polypeptide.

The selection of the vector is guided by the type of host cells which is used for RNA or protein synthesis.

One preferred vector is a vector appropriate for expression in *E. coli*, and more particularly a plasmid containing at least one *E. coli* replication origin and a selection gene of resistance to an antibiotic, such as the $Ap^R$ (or *bla*) gene.

In one embodiment, the cellular concentration of α subunit of RNA polymerase is increased by overexpressing in the host cell, a gene encoding an α subunit of RNA polymerase.

Preferably, a gene encoding an α subunit of RNA polymerase is a gene from *E. coli, T. maritima, T. neapolitana or T. thermophilus.*

For example, the host cell can comprise, integrated in the genome, an expression cassette comprising a gene encoding an α subunit of RNA polymerase under the control of an inducible or derepressible promoter, while the expression of the other subunits remains unchanged.

An expression cassette comprising a gene encoding an α subunit of RNA polymerase can also be incorporated into the expression vector comprising the DNA template of the invention, or into a second expression vector.

For example, the expression cassette comprises the *E. coli* gene *rpoA,* under the control of a T7 phage promoter.

In a preferred embodiment, the concentration of α subunit in a cellular system is increased by induction of the expression of an additional copy of the gene encoding α subunit of RNA polymerase while expression of the other subunits remains unchanged.

In another specific and preferred use of said DNA template of the invention, said system enabling RNA or polypeptide synthesis from the DNA template according to the invention, is a cell-free system comprising a bacterial cell-free extract.

For cell-free synthesis, the DNA template can be linear or circular, and generally includes the sequence of the Open Reading Frame corresponding to the RNA or protein of interest and sequences for transcription and translation initiation. Lesley *et al*., (1991) optimised the Zubay (1973) *E. coli* S30 based-method for use with PCR-produced fragments and other linear DNA templates by preparing a bacterial extract from a nuclease-deficient strain of *E. coli*. Also, improvement of the method has been described by Kigawa *et al*. (1999) for semi-continuous cell-free production of proteins.

When a cell-free extract is used for carrying out the method of the invention, the concentration of α subunit of RNA polymerase is preferably increased by adding purified α subunit of RNA polymerase to the cell free extract. When using the DNA templates of the invention, it is indeed preferred that no other subunits of RNA polymerase are added to the cell-free extract, so that the stoechiometric ratio of α subunit / other subunits is increased in the cell-free extract in favour to the α subunit. Preferably, said purified α subunit is added in a cell-free extract, more preferably a bacterial cell-free extract, to a final concentration comprised between 15 μg/ml and 200 μg/ml.

Purified α subunit of RNA polymerase can be obtained by the expression in cells of a gene encoding an α subunit of RNA polymerase and subsequent purification of the protein. For example, α subunit of RNA polymerase can be obtained by the expression of the *rpoA* gene fused in frame with a tag sequence in *E. coli* host cells, said fusion enabling convenient subsequent purification by chromatography affinity.

The term "bacterial cell-free extract" as used herein defines any reaction mixture comprising the components of transcription and/or translation bacterial machineries. Such components are sufficient for enabling transcription from a deoxyribonucleic acid to synthesize a specific ribonucleic acid, i.e mRNA synthesis. Optionally, the cell-free extract comprises components which further allow translation of the ribonucleic acid encoding a desired polypeptide, i.e polypeptide synthesis.

Typically, the components necessary for mRNA synthesis and/or protein synthesis in a bacterial cell-free extract include RNA polymerase holoenzyme, adenosine 5'triphosphate (ATP), cytosine 5'triphosphate (CTP), guanosine 5'triphosphate (GTP), uracyle 5'triphosphate (UTP), phosphoenolpyruvate, folic acid, nicotinamide adenine dinucleotide phosphate, pyruvate kinase, adenosine, 3', 5'-cyclic monophosphate (3',5'cAMP), transfer RNA, amino-acids, aminoacyl tRNA-synthetases, ribosomes, initiation factors, elongation factors and the like. The bacterial cell-free system may further include bacterial or phage RNA polymerase, 70S ribosomes, formyl-methionine synthetase and the like, and other factors necessary to recognize specific signals in the DNA template and in the corresponding mRNA synthesized from said DNA template.

A preferred bacterial cell-free extract is obtained from *E. coli* cells.

A preferred bacterial cell-free extract is obtained from genetically modified bacteria optimised for cell-free RNA and protein synthesis purposes. As an example, *E. coli* K12 A19 is a commonly used bacterial strain for cell-free protein synthesis.

The efficiency of the synthesis of proteins in a cell-free synthesis system is affected by nuclease and protease activities, by codon bias, by aberrant initiation and/or termination of translation. In an effort to decrease the influence of these limiting factors and to improve the performance of cell-free synthesis, specific strains can be designed to prepare cell-free extract lacking these non-desirable properties.

It has been shown in the present invention that *E. coli* BL21Z which lacks Lon and OmpT major protease activities and is widely used for *in vivo* expression of genes, can also be used advantageously to mediate higher protein yields than those obtained with cell-free extracts from *E. coli* A19. Thus, one specific embodiment comprises the use of cell-free extracts prepared from *E. coli* BL21Z.

In bacterial cell-free systems, a major part of the synthesized mRNA are unprotected against hydrolysis and can be subjected to degradation by the RNase E-containing degradosome present in bacterial cell-free extracts. Truncation mutations in the C-terminal or in the internal part of RNase E stabilise transcripts in *E. coli* cells. Thus, cell-free extracts from *E. coli* strains which are devoid of RNaseE activity and also protease activity, can be used in cell-free systems for RNA or protein synthesis. Such a strain, *E. coli* BL21 (DE3) Star, is commercially available from Invitrogen.

The RecBCD nuclease enzymatic complex is a DNA reparation system in *E. coli* and its activation depends upon the presence of Chi sites (5'GCTGGTGG3') (SEQ ID NO: 22) on *E. coli* chromosome. Therefore, a *recBCD* mutation can be introduced in *E. coli* host cells in order to decrease the degradation of DNA templates in a cell-free system.

When several codons code for the same amino acid, the frequency of use of each codon by the translational machinery is not identical. The frequency is increased in favor to preferred codons. Actually, the frequency of use of a codon is species-specific and is known as the codon bias. In particular, the *E. coli* codon bias causes depletion of the internal tRNA pools for AGA/AGG (*argU*) and AUA (*ile Y*) codons. By comparing the distribution of synonymous codons in ORFs encoding a protein or RNA of interest and in the *E. coli* genome, tRNA genes corresponding to identified rare codons can be added to support expression of genes from various organisms. The *E. coli* BL21 Codon Plus-RIL strain, which contains additional tRNA genes modulating the *E. coli* codon bias in favor to rare codons for this organism, is commercially available from Stratagene and can be used for the preparation of cell-free extract.

Also, improved strains can be used to prevent aggregation of synthesized proteins which can occur in cell-free extracts.

For example, it is well documented that chaperonines can improve protein solubility by preventing misfolding in microbial cytoplasm. In order to decrease a possible precipitation of proteins synthesized in a cell-free system, *groES-groEL* region can be cloned in a vector downstream an inducible or derepressible promoter and introduced into a *E. coli* host cell.

Both, protein yield and protein solubility, can further be improved in the presence of homologous or heterologous GroES/GroEL chaperonines in cell-free extracts, prepared from modified *E. coli* strains, whatever is the selected expression system.

In another embodiment, the cell-free extract is advantageously prepared from cells which overexpress a gene encoding α subunit of RNA polymerase.

Preferred host cells and plasmids used for overexpression of a gene encoding α subunits have been described previously.

Indeed, cell-free extracts prepared from cells overexpressing RNA polymerase α subunit provide improved yield of protein synthesis.

In a preferred embodiment, cell-free extracts are prepared from *E. coli* strains such as the derivatives of BL21 strain or the *E. coli* XA 4 strain, overexpressing the *rpoA* gene.

One advantage of the present embodiment is that the overexpression of α subunit of RNA polymerase is endogeneous and does not need the addition of an exogenous α subunit of RNA polymerase to the reaction mixture. It makes the experimental performance easier and decreases the total cost of *in vitro* protein synthesis.

It is known in the art that adding purified RNA polymerase may improve the yield of protein synthesis. For example, purified T7 polymerase can be added to the reaction mixture when carrying out cell-free synthesis using a T7 phage promoter. Preferably; adding purified RNA thermostable polymerase, preferably *T. thermophilus,* in combination with the addition of purified α subunit of RNA polymerase and using bacterial promoter, enables much better yield than with the use of T7 polymerase promoter system.

Thus, in a preferred embodiment, purified thermostable RNA polymerase, preferably from *T. thermophilus,* is added into a bacterial cell-free extract.

The isolation according to the invention of strong bacterial promoters of bacterial pathogens also provides new approaches for the screening of antibacterial agents which inhibit transcription by binding to strong promoters of said pathogens.

Accordingly, another object of the invention is the use of said isolated nucleic acid having strong bacterial promoter activity for the screening of antibacterial agents which bind to said isolated nucleic acid having strong bacterial promoter activity.

The examples below illustrate some specific embodiments of the invention. Especially, the examples illustrate the identification and isolation of bacterial strong promoters from *T. maritima.*

## LEGENDS OF THE FIGURES

**Figure 1: A single-strand sequence of putative *Thermotoga maritima* promoter regions amplified by PCR and the ribosome-binding site used for translation of a reporter gene.**

A putative UP-element is shown in *italic*; putative -35 and -10 sites are underlined; promoter regions putative by algorithm are shown in bold. A sequence carrying Shine-Dalgarno site GGAGG was placed 12 - 15 nucleotides downstream the putative -10 site in the corresponding T. *maritima* promoter. The Shine-Dalgarno site and the ATG initiation codon used for the *B. stearothermophilus argC* reporter-gene are shown in bold and underlined; additional sequences used to extend the distance between -10 site and Shine-Dalgarno site in tRNAthr1 and TM1016 sequences are shown by lowercase.

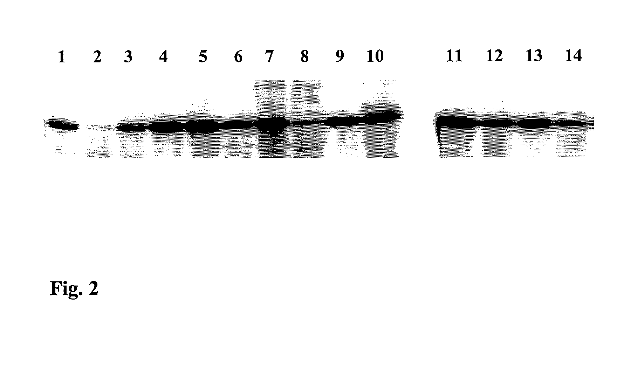

**Figure 2: Autoradiogram of ArgC reporter protein synthesis *in vitro* from DNA templates carrying *T. maritima* promoter regions.**

The *B. stearothermophilus argC* reporter gene was expressed from putative T. *maritima* promoter regions or a P*tac* promoter *in vitro* using *E. coli* S30 extracts. 50 ng of each PCR amplified DNA template was used for *in vitro* protein synthesis.

Lane 1 - P*tac* (control); lane 2 - P*TM0032;* lane 3 - P*TM0373*; lane 4 - P*TM0477*; lane 5 - P*TM 1016*; lane 6 - P*TM1067*; lane 7 - P*TM1271*; lane 8 - P*TM1272*; lane 9 - P*TM1429*; lane 10 - P*TM1490*; lane 11 - P*TM1667*; lane 12 - P*TM1780*; lane 13 - P*TMtRNAser1*; lane 14 - P*TMtRNAthr1*.

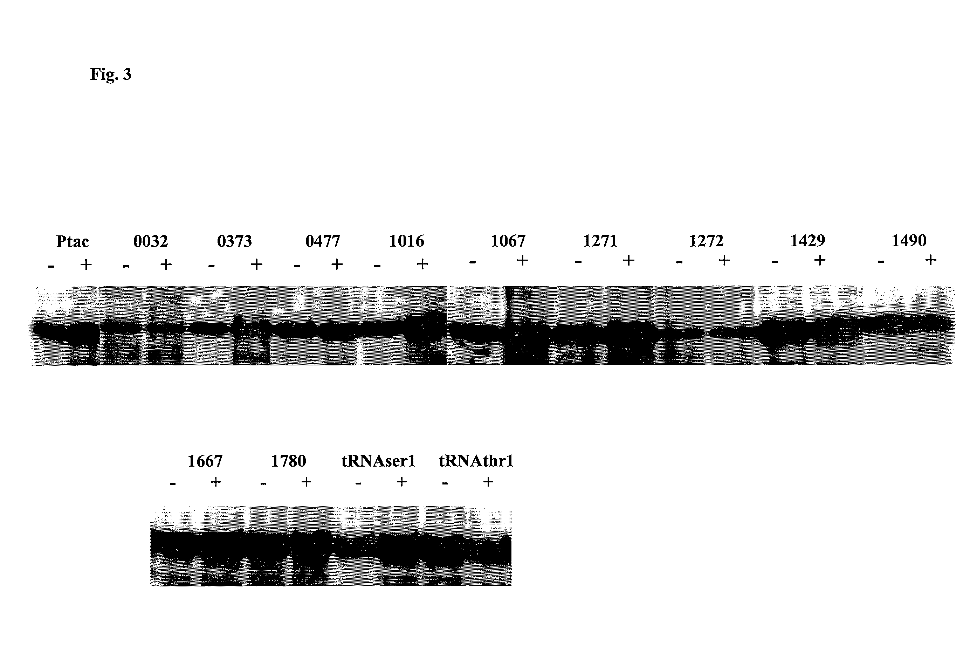

**Figure 3: Autoradiogram of ArgC reporter protein synthesis from DNA templates carrying *T. maritima* promoter regions in the absence and in the presence of α subunit of *T. maritima* RNA polymerase.**

The *B. stearothermophilus argC* reporter gene was expressed from putative *T. maritima* promoter regions or a P*tac* promoter in the absence (-) or in the presence (+) of 800 nM purified *T. maritima* RNA polymerase α subunit. 50 ng of each PCR amplified DNA template was used for *in vitro* protein synthesis.

**Figure 4: Autoradiogram of *T. maritima* ArgG synthesis in the presence and in the absence of α subunit of *T. maritima* RNA polymerase.**

A 1633 bp *T. maritima* DNA region covering the promoter P*argG* and the *argG* gene was amplified by PCR and used for the ArgG protein synthesis *in vitro* in the absence (lane 1) or in the presence of *T. maritima* RNA polymerase α subunit, 400 nM (lane 2) and 800 nM (lane 3).

**Figure 5: Alignment of strong promoter sequences from T. *maritima*.**

The sequence logo for the *T. maritima* UP element and -35 site was generated with a software at *http://www.bio.cam.ac.uk/seqlogo/logo.cgi*. An additional N is included into the *E. coli* UP consensus just before -35 since the residue at this position is not taken into consideration for strong promoter activity in this species.

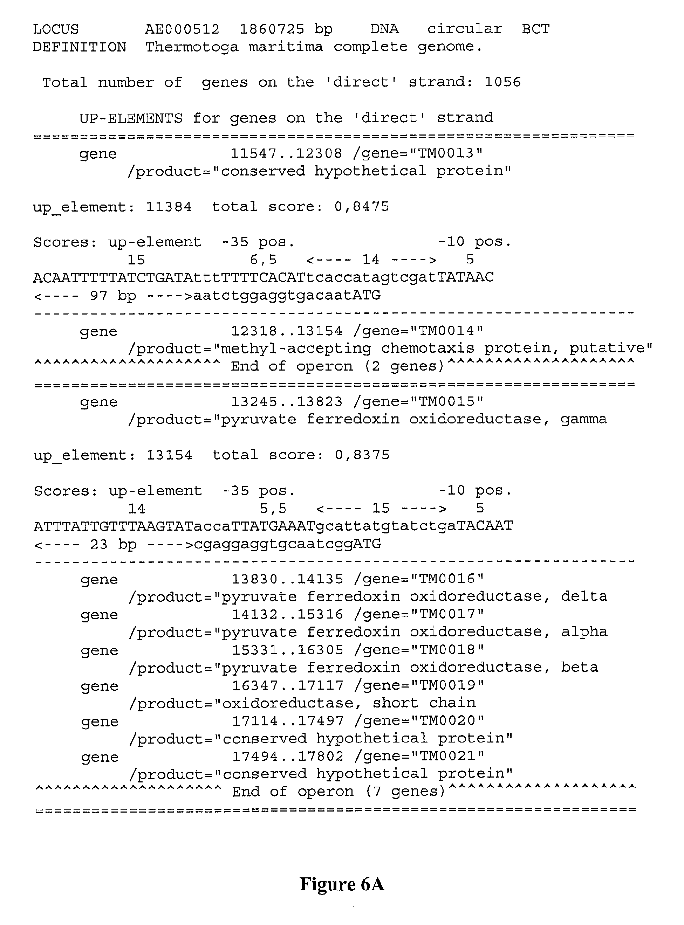

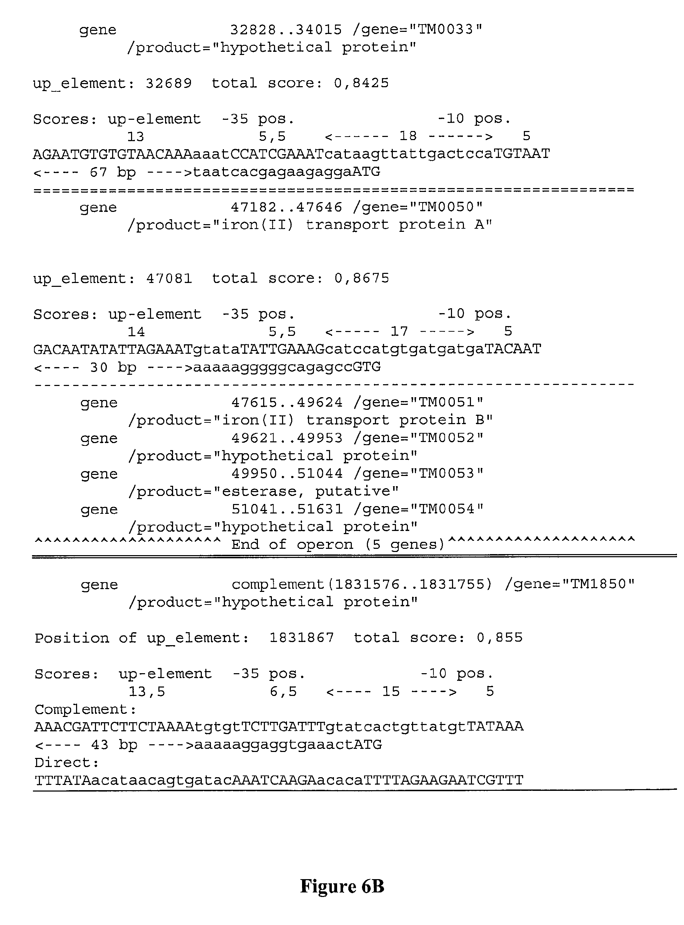

**Figure 6: Text file presentation of putative strong promoters**

The data are shown in the Text file with the list of selected strong promoters in the genome with additional information on the operon structure.

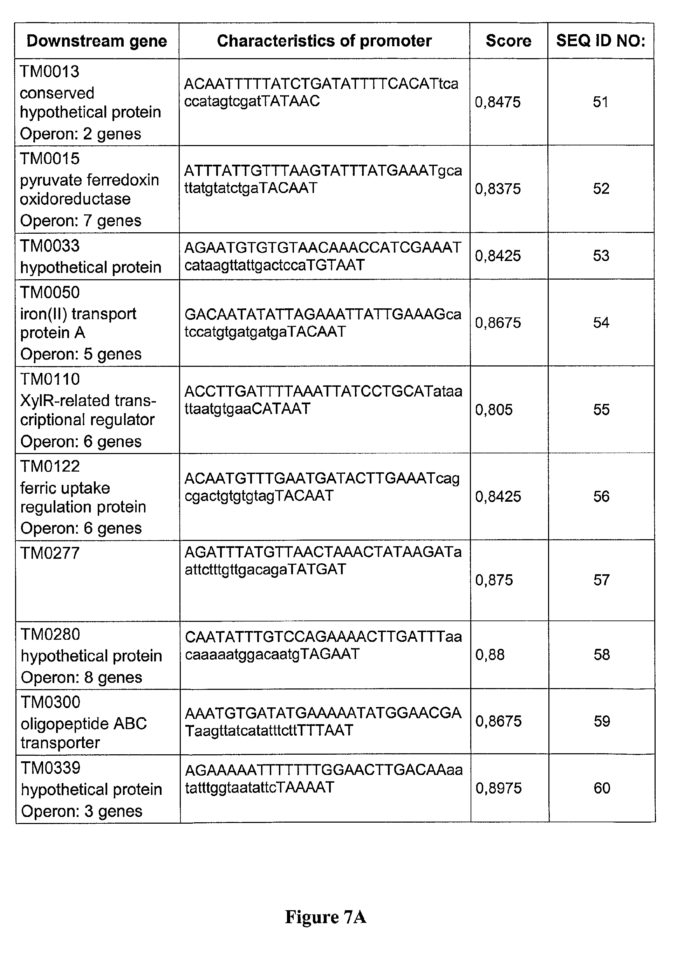

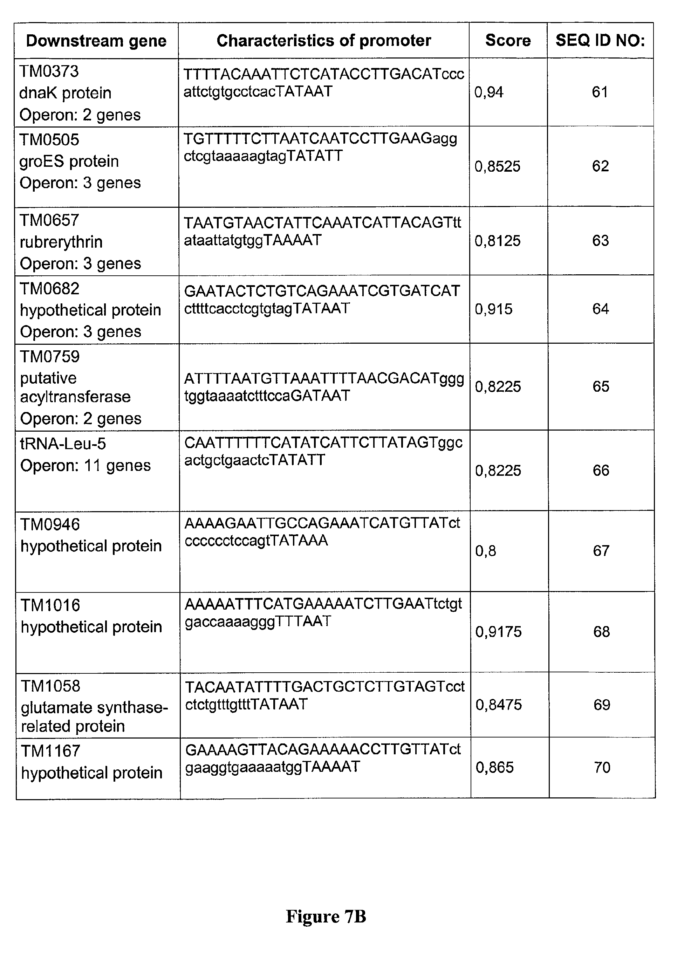

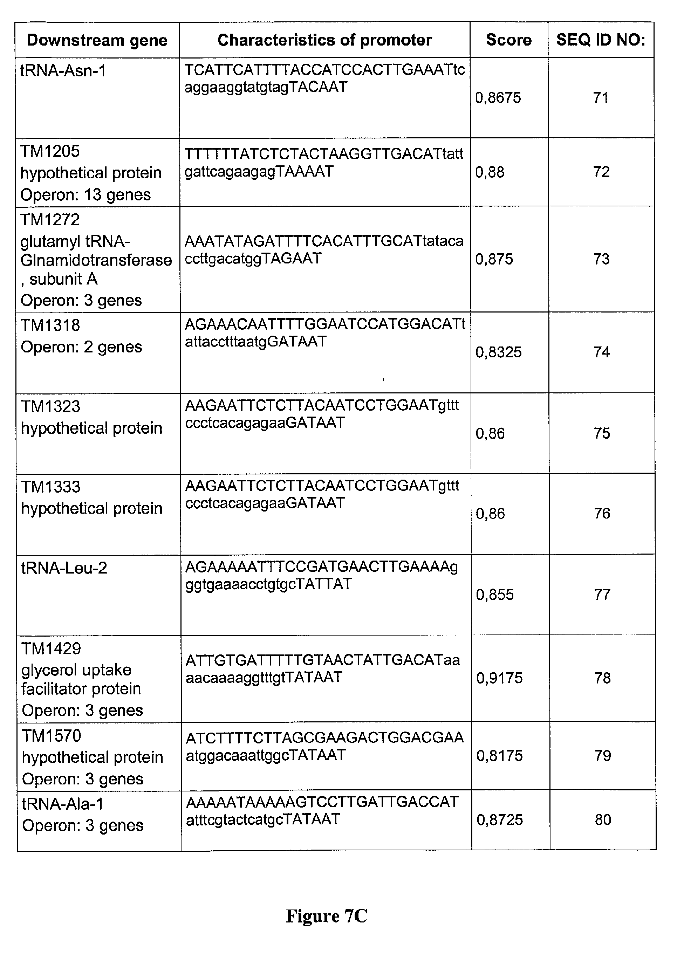

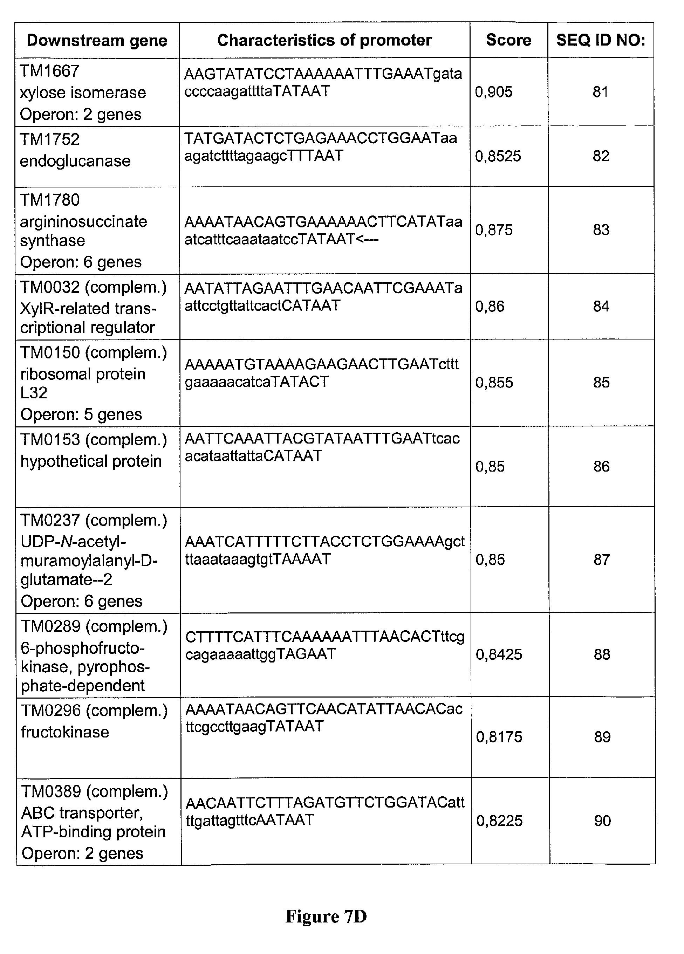

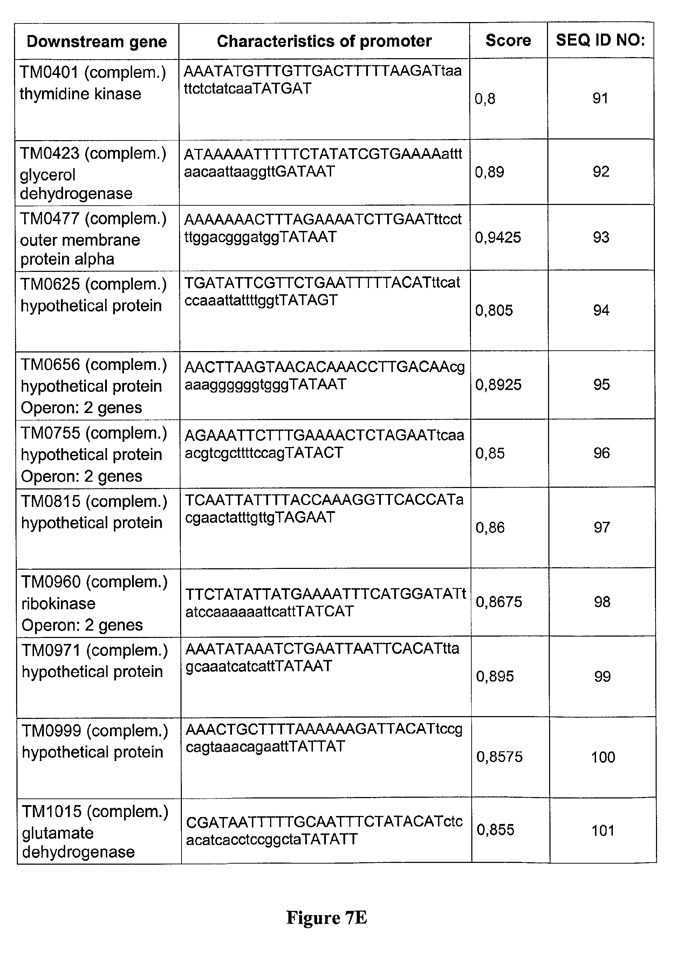

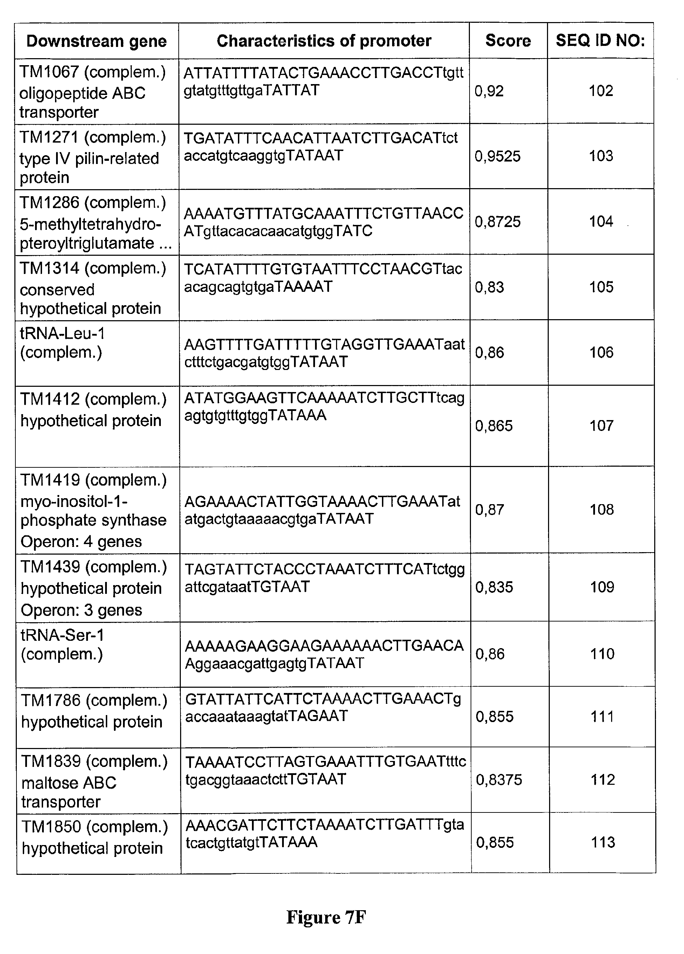

**Figure 7: Word form presentation of putative strong promoters in *T. maritima* genome**

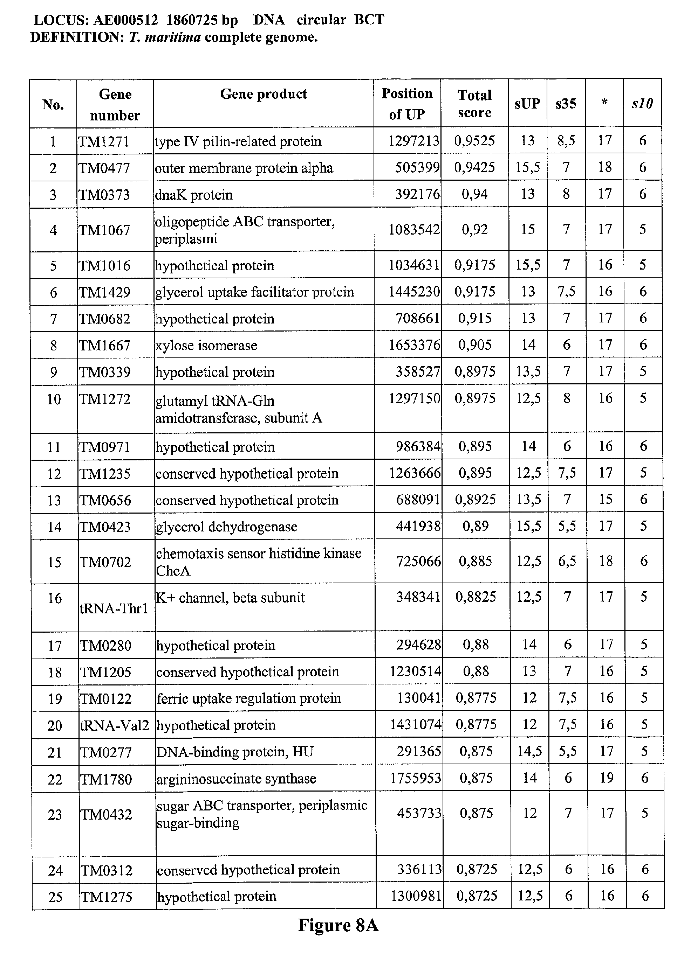

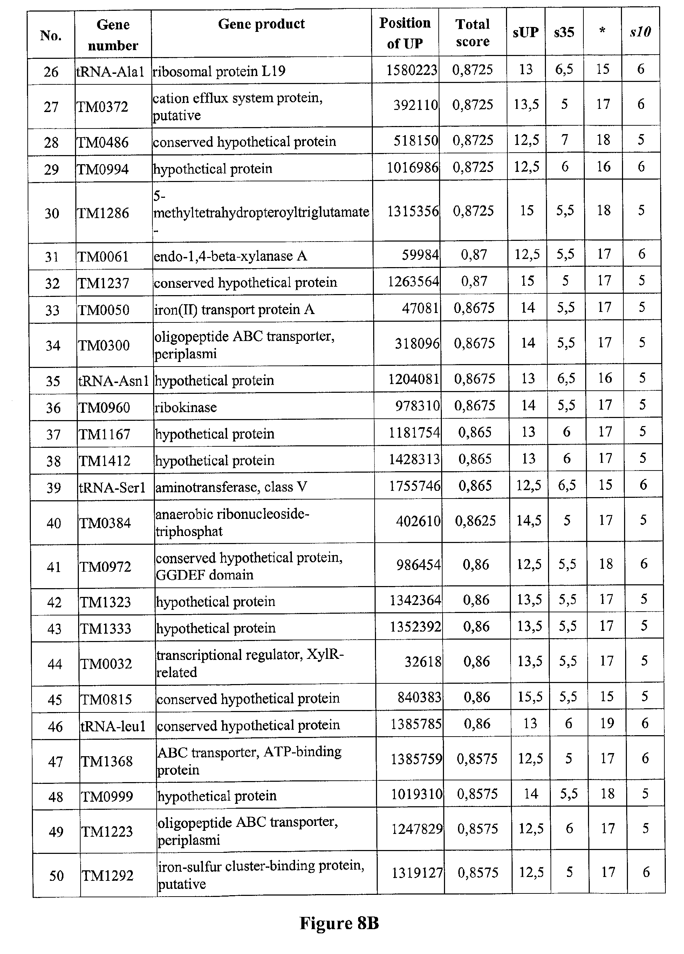

**Figure 8: Excell form presentation of putative strong promoters in T.maritima genome**

The data are shown with the list of putative strong promoters ordered by their total scores.

## EXAMPLES

### A. Material and Methods

### A.1 Algorithm for searching putative strong promoters in microbial genomes

A single-strand DNA can be described as a sequence over the four-symbol alphabet {a, c, g , t}, in which a is Adenine, c is Cytosine, g is Guanine and t is Thymine. The DNA length can be measured in nucleotides (nt) for a single-strand molecule or in base pairs (bp) for a double-strand one.
In the present invention, a new algorithm "STRONG_PROMOTERS_SEARCH" was developed for searching strong promoters in DNA sequences. Thanks to its flexibility, the algorithm can be applied to any microbial genome.

In the present example, a strong bacterial promoter sequence is a DNA region of a size from 44 to 66 bp located upstream the transcription start site of a given gene (coding for protein or tRNA or rRNA sequence), recognized by RNA polymerase holoenzyme containing a major σ factor, and which includes three special nucleotide subregions:

1) an UP-element, which is a 17 nt prefix of the strong promoter and has the following consensus pattern "aaaWWtWttttNNNaaa", where "W" stands for the pair of symbols "a" and "t" and "N" denotes any of four symbols "a", "t", "c", and "g";
2) -35 site, which is located downstream of the UP-element at the distance of 0 - 5 nt and has the following consensus pattern "tcttgacat" (underlining marks a commonly used pattern);
3) -10 site, which is located downstream of -35 site at the distance of 14 - 20 nt and has the following consensus pattern "tataat".

The algorithm uses similarity scores between two sequences, which is the sum of coincidence rates of symbols in the corresponding positions: the equality rate is 1 whereas the nonequality rate is lower than 1 and is determined empirically for each pair of symbols. Therefore, the similarity score of each consensus pattern for any compared sequence varies from 0 to the corresponding length, namely 17 for UP-element, 9 for -35 site and 6 for -10 site.

The algorithm takes as input:

1) the name of a genome file in the format GenBank;
2) three parameters of scores: *scUP,* sc35 and *sc10* determining the minimal acceptable value of similarity between UP-element, -35 site and -10 site respectively and the corresponding consensus pattern. Their values 11, 5 and 4 were chosen empirically and are predefined by default, however other values can be input before starting the program.

For each gene in the input genome file, the algorithm runs as follows:

1) first, it extracts an upstream DNA region, namely 300 bp upstream of the corresponding open reading frame or gene coding for tRNA or rRNA;
2) next, it searches for a strong promoter within this region checking a subregion of the length 70 bp. The algorithm determines the similarity score *sUP* for the 17 nt prefix with the UP-element consensus pattern (the maximal possible value of *sUP* is 17) in each identified subregion. If *sUP* is greater or equal to the given minimal score *scUP*, then the algorithm checks whether there is an appropriate -35 site downstream of UP-element. In order to obtain the -35 site with the best possible score *s35*, it uses a special kind of a dynamic programming alignment algorithm, which prohibits any two subsequent insertions or deletions in the -35 consensus pattern and in the chosen DNA subsequence (the maximal possible value of *s35* is 9). If *s35* is greater or equal to the given minimal score *sc35*, then the algorithm checks whether there is an appropriate -10 site downstream of -35 site by checking first the distance of 17 nt from the end of -35 site, then by subsequent checking distances of 18, 16, 19, 15, 20 and 14 nt (the maximal possible value of *s10* is 6). If *s10* is greater or equal to the given minimal score *sc10*, then the corresponding subregion is included into the list of strong promoters of corresponding genes.
3) For all found strong promoter sequences of each gene, a normalized total score is computed and the best one is output. The normalized total score *tot sc* is defined as follows:

$$tot_sc = 0.30*nsc_up + 0.25*nsc_35 + 0.25*nsc_10 + 0.2*nsc_dist,$$

where normalized scores *nsc_up, nsc_35, nsc_10* are defined by the formulas:

$$nsc_up = 1 - (17 - sUP)/20,$$

$$nsc_35 = 1 - (9 - s35)/10,$$

$$nsc_10 = 1 - (6 - s10)^2/10,$$

and the values of the normalized distance score *nsc_dist* are defined in Table 1.

The formulas for *nsc_up, nsc_35* and *nsc_10* reflect the inexact matching for different subregions. Since -10 site is highly conserved as "tataat" sequence, and then the penalty for each mismatching should be rather high. For example, for 2 mismatches the penalty is $(6 - 4)^2/10=0,4$ for -10 site, whereas it is $(9 - 6)/10=0.3$ for -35 site and $(17 - 15)/20=0,1$ *for* UP-element.

The coefficients 0.30, 0.25 and 0.2 used in the first formula, reflect the relative importance of corresponding subregion for the evaluation of the total score of a strong promoter. They are chosen empirically taking into account the equal significance of -10 and -35 sites, lower significance of the distance between them and higher significance of UP-element. The rate of similarity for each subregion can be modulated by increasing or decreasing the coefficients. However, the set of strong promoters recognized by the developed algorithm doesn't essentially depend on small changes of these coefficients.

Algorithm "STRONG_PROMOTERS_SEARCH" produces the results in 3 forms:

1) Text-form table with the list of all strong promoters of a genome with additional information on the operons structure (example in figure 6);
2) Word-form table with the list of strong promoters (example in figure 7);
3) Excel-form table with the list of strong promoters ordered by their total scores (example in figure 8).

### A.2 Cloning the *rpoA* gene from *T. maritima*

Chromosomal DNA of the *T. maritima* MSB8 strain was isolated as described previously (Dimova *et al*., 2000). A sequence corresponding to the *rpoA* gene of the RNA polymerase α subunit of *T. maritima* (Nelson *et al*., 1999) was amplified on a chromosomal DNA by PCR and two oligonucleotide primers 5'CCATGGCTATAGAATTTGTGATACCAAAAAAATTGAAGGTG (SEQ ID NO:17) containing the *Nco*I site and 5'GTCGACTTCCCCCTTCCTGAGCTCAAG (SEQ ID NO:18) containing the *Sal*I site. The amplified DNA fragment was digested by *Nco*I and *Sal*I and cloned in frame with the C-terminal His-tag sequence of the pET21d+ vector digested by *Nco*I and *Xho*I giving rise to pETrpoA. The cloned DNA region with junction sites was verified by automatic DNA sequencing.

### A.3 Purification of the recombinant RNA polymerase α subunit of *T. maritima*

Overexpression of the cloned *T. maritima rpoA* gene was performed in *E. coli* BL21(DE3) (Novagen) by the addition of IPTG (1 mM) to a culture grown up to $OD_{600nm}$ = 0.8 and further incubation of cells at 30°C for 4 hours. The His-tagged RNA polymerase α subunit was next purified from the IPTG-induced culture on a Ni-NTA column by affinity chromatography following a recommended protocol (Qiagen). The purified RNA polymerase α subunit samples were quantified with Lab-on-chip Protein 200 plus assay kit with 2100 Bioanalyzer (Agilent Technologies).

### A.4 Construction of DNA templates for *in vitro* synthesis of a reporter protein ArgC

The putative promoter regions of *T. maritima* by the developed algorithm were amplified on chromosomal DNA by PCR using a couple of oligonucleotide primers corresponding to sequences located upstream and downstream of each promoter region. The *tac* promoter region was also amplified from the plasmid pBTac2 (Bohringer & Mannheim). This chimeric promoter consisting of the native P*trp* and P*lac* promoters was used as a control strong promoter for comparative analysis of putative *T. maritima* promoters. Primers used for amplification of promoter regions are described in the following Table 2.

**Table 2. Oligonucleotide primers used for amplification of *T. maritima* promoter regions.**

The sequence used for overlapping between promoter and the reporter *argC* gene is shown in bold.

| Primers | Oligonucleotide séquence | SEQ ID NO: |
|---|---|---|
| Ptac up | 5'GCGCCGACATCATAACGG | 23 |
| Ptacdown | 5'**CATATGTTCCCCCTCC**TCACAATTCCACACATTATACC | 24 |
| P0032 up | 5'GCTCCTTGGAAAGAGCATCG | 25 |
| P0032 down | 5'**CATATGTTCCCCCTCC**TACTCATTTTTTATTATGAG | 26 |
| P0373 up | 5'ATATTCGATTTCCCTCATATTTAGG | 27 |
| P0373 down | 5'**CATATGTTCCCCCTCC**TCTCATCCATGAAAAATTATAG | 28 |
| P0477 up | 5'GAGAGTTGGAAAGAGGAAG | 29 |
| P0477 down | 5'**CATATGTTCCCCCTCC**TTAAATCCTGTGGTGATTAT | 30 |
| P1016 up | 5'CCATATCGTTTACCTATTG | 31 |

| Primers | Oligonucleotide séquence | SEQ ID NO: |
|---|---|---|
| P1016 down | 5'**CATATGTTCCCCCTCC**CCCGTATGGCTATATAT TAAACCCTTTTGG | 32 |
| P1067 up | 5'GGGGTTGTAAGCAAAAGG | 33 |
| P1067 down | 5'**CATATGTTCCCCCTCC**CTTGAAGTTATCAATATA ATATC | 34 |
| P1271 up | 5'CGGTTTGTCTTTGAGACGAAT | 35 |
| P1271 down | 5'**CATATGTTCCCCCTCC**ATTTTCACATTTTGCATT ATAG | 36 |
| P1272 up | 5'CCCGCTCTCTTTCTCATT | 37 |
| P1272 down | 5'**CATATGTTCCCCCTCC**ATTAAAATCTTGACATTC TACC | 38 |
| P1429 up | 5'GAAAGAAGACGTGGAAAG | 39 |
| P1429 down | 5'**CATATGTTCCCCCTCC**TATGCCTCGATGTGAAT TATAAC | 40 |
| P1490 up | 5'GCCAGGATAAAGACCATTC | 41 |
| P1490 down | 5'**CATATGTTCCCCCTCC**ACTGTCTTGTCCATTTTA TC | 42 |
| P1667 up | 5'CCTCTCTGAGCTCTTCTA | 43 |
| P1667 down | 5'**CATATGTTCCCCCTCC**TTTTTCTATCAATCAAT | 44 |
| P1780 up | 5'GATATTCATAAACACGAA | 45 |
| P1780 down | 5'**CATATGTTCCCCCTCC**GTTCTTGATAGCATAATT ATAGG | 46 |
| Prna ser1 up | 5'CATCTTTGCACTTTTCG | 47 |
| Prna ser1down | 5'**CATATGTTCCCCCTCC**ACACCAGAAAAATATTA TACAC | 48 |
| Prna thr1 up | 5'TACCAAGGTACGTGGTGA | 49 |
| Prna thr1 down | 5'**CATATGTTCCCCCTCC**CCCGTATGTGCCCGTAT GTGTGGTTATTTTAACACACG | 50 |

The first PCR amplification step was performed with Platinum *Pfx* DNA polymerase (Invitrogen). Next, the *B. stearothermophilus argC* gene (Sakanyan *et al*., 1990; 1993) was used as a reporter to evaluate the strength of isolated promoter regions. In order to increase gene expression an original SD-site of *argC* was modified from TGAGG to GGAGG. The *argC* gene was amplified by PCR using primers argC8-deb (5'-GGAGGGGGAACATATGATGAA) (SEQ ID NO:19) and argCfin-pHav2 (5'-GGACCACCGCGCTACTGCCG) (SEQ ID NO:20) and the obtained DNA fragment was fused downstream of the 13 studied promoters by the "overlapping extension" method (Ho *et al.,* 1989). For each construction, the amplified DNAs for a given promoter region of *T. maritima* and the *B. stearothermophilus argC* gene region were combined in a subsequent fusion PCR product using two flanking primers by annealing of the overlapped ends to provide a full-length recombinant DNA template. The overlapping region is shown in bold in the used primer sequences (see Table 2). The second PCR reaction was carried by Goldstar Taq DNA polymerase (Eurogentec). The DNA templates obtained by overlapping extension were quantified by Lab-on chip DNA 7500 assay kit with 2100 Bioanalyzer (Agilent Technologies) by injecting 1 µl of a PCR product.

### A.5 Preparation of cell-free extracts

A strain *E. coli* BL21 (DE3) Star RecBCD was used for the preparation of cell-free extracts by the method of Zubay (1973) with modifications as follow:

Cells were grown at 37°C to OD=0.8, harvested by centrifugation and washed twice thoroughly in ice-cold buffer containing 10 mM Tris-acetate pH 8.2, 14 mM Mg-acetate, 60 mM KCl, 6 mM β-mercaptoethanol. Then, cells were resuspended in a buffer containing 10 mM Tris-acetate pH 8.2, 14 mM Mg-acetate, 60 mM KCl, 1 mM dithiotreitol and disrupted by French press (Carver, ICN) at 9 tonnes (≈ 20.000 psi). The disrupted cells were centrifuged at 30.000 g at 4°C for 30 min, the pellet was discarded and the supernatant was centrifuged again. The clear lysate was added in a ratio 1 : 0.3 to the preincubation mixture containing 300 mM Tris-acetate at pH 8.2, 9.2 mM Mg-acetate, 26 mM ATP, 3.2 mM dithiotreitol, 3.2 mM L-amino acids and incubated at 37°C for 80 min. The mixed extract solution was centrifuged at 6000 g at 4°C for 10 min, dialysed against a buffer containing 10 mM Tris-acetate pH 8.2, 14 mM Mg-acetate, 60 mM K-acetate, 1 mM dithiotreitol at 4°C for 45 min with 2 changes of buffer, concentrated 2-4 times by dialysis against the same buffer with 50% PEG-20.000, followed by additional dialysis without PEG for 1 hour. The obtained cell-free extract was distributed in aliquots and stored at -80°C.

### A.6 Cell-free protein synthesis by coupled transcription-translation reaction

The coupled transcription-translation reaction was carried out as described by Zubay (1973) with some modifications. The standard pre-mix contained 50 mM Tris-acetate pH 8.2, 46.2 mM K-acetate, 0.8 mM dithiotreitol, 33.7 mM NH4-acetate, 12.5 mM Mg-acetate, 125 µg/ml tRNA from *E. coli* (Sigma), 6 mM mixture of CTP, GTP and TTP, 5.5 mM ATP, 8.7 mM CaCl2, 1.9 % PEG-8000, 0.32 mM L-amino acids, 5.4 µg/ml folic acid, 5.4 µg/ml FAD, 10.8 µg/ml NADP, 5.4 µg/ml pyridoxin, 5.4 µg/ml para-aminobenzoic acid. Pyruvate was used as the energy regenerating compound (Kim and Swartz, 1999) by addition of 32 mM pyruvate in 6.7 mM K-phosphate pH 7.5, 3.3 mM thiamine pyrophosphate, 0.3 mM FAD and 6 U/ml pyruvate oxidase (Sigma). Typically, 50 ng of linear PCR-amplified DNA template was added to 25 µl of a pre-mix containing all the amino acids except methionine, 10 µCi of [$\alpha^{35}$S]-L-methionine (specific activity 1000 Ci/mmol, 37 TBq/mmol, Amersham-Pharmacia Biotech) and *E. coli* S30 cell-free extracts. The reaction mixture was then incubated at 37°C for 90 min. The purified α subunit of *T. maritima* RNA polymerase was added to the reaction mixture at different concentrations. The protein samples were treated at 65° C for 10 min and then quickly centrifuged. The supernatant was precipitated with acetone and used for protein separation on SDS-PAGE, gels were treated with an amplifier solution (Amersham-Pharmacia Biotech), fixed on a 3 MM paper by vacuum drying and the radioactive bands were visualized by autoradiography using BioMax MR film (Kodak). Quantification of cell-free synthesized proteins was performed by counting radioactivity of $^{35}$S-labeled ArgC protein with a PhosphorImager 445 SI (Molecular Dynamics).

## B. EXAMPLES

### B.1 Example 1: Identification of strong promoters in *T. maritima*

As example, the algorithm of "STRONG_PROMOTERS_SEARCH" was used for searching strong promoters in the *T. maritima* genome. The data are shown in the 3 forms, namely:

1) in the Text file with the list of selected strong promoters in the genome with additional information on the operon structure (Figure 6A-6B). 33 putative strong promoters identified on a "direct" strand, whereas 30 putative strong promoters were identified on a "complementary" strand.

2) in the Word form with the list of the putative strong promoters (Figure 7A-7F);
3) in the Excel form with the list of putative strong promoters ordered by their total scores (Figure 8A-8B).

**B.2 Example 2 : Putative promoter sequences of *T. maritima sequences* exhibit a high activity *in vitro***

To confirm the presence of functional promoters in the putative *T. maritima* sequences and to measure the activity of these potential promoters, 13 putative promoter sequences (Figure 1) were fused to the *B. stearothermophilus argC* reporter-gene coding for *N*-acetyl glutamylphosphate reductase. The fused DNA fragments were next used as templates for performing ArgC synthesis *in vitro*, namely in the coupled *E. coli* transcription-translation system. Eight sequences were selected from the first 10 selected putative strong promoters shown with a score higher than 0.8975 in figure 8. Five others were selected from promoters displaying lower score.The strong P*tac* promoter, which has a score of 0.8225 was fused to the reporter gene and used as a reference for comparison with the protein yield provided from *T. maritima* promoters.
50 ng of such homogen DNA templates, as qualified and quantified by the biochip method, were included into the reaction mixture and protein synthesis was initiated by the addition of S30 extracts.
All *T. maritima* sequences promoted ArgC synthesis as indicative of a presence of functional promoters (Figure 2). Moreover, all promoter-carrying DNA templates, except for the TM0032 and TM1272 genes, provided higher protein synthesis as compared to the P*tac* promoter (the protein yield from the latter was taken as 1 for reference). The 13 selected *T. maritima* promoters increased the protein yield from 0.5-fold to 2.7-fold (average data from 3 independent experiments) as compared to the P*tac* promoter (Table 3).

Table 3

| ***T. maritime* promoter strength *in vitro* and the effect of *T. maritime* RNA polymerase α subunit on ArgC reporter-protein synthesis** | | | | | |
|---|---|---|---|---|---|
| **Promoter Name** | **sUP** | **Total score** | **Protein** | **Comparative promoter strength** | **Effect of α subunit** |
| 1271 | 13 | 0.9525 | Pilin related protein | 2.2 | 1.2 |
| 0477 | 15.5 | 0.9425 | Outer membrane protein α | 2.7 | 2.6 |
| 0373 | 13 | 0.9400 | DnaK | 2.1 | 1.5 |
| 1067 | 15 | 0.9200 | ABC transporter periplasmic | 1.6 | 1.7 |
| 1016 | 15.5 | 0.9175 | Hypothetical protein | 2.5 | 1.2 |
| 1429 | 13 | 0.9175 | Glycerol uptake facilitator | 2.4 | 1.2 |
| 1667 | 14 | 0.9050 | Xylose isomerase | 2.2 | 1.2 |
| 1272 | 12.5/14 .5 | 0.8975 | Glutamyl tRNA Gln amidotransferase | 0.9 | 1.7 |
| rna thr1 | 12.5 | 0.8825 | tRNA thr1 | 1.7 | 1.2 |
| 1780 | 14 | 0.8750 | ArgG | 2 | 1.2 |
| rna ser1 | 12.5 | 0.8625 | tRNA ser1 | 2.5 | 1.3 |
| 1490 | 12.5 | 0.8450 | Ribosomal protein L14 | 2.1 | 1.2 |
| 0032 | 13.5 | 0.8600 | XylR | 0.5 | 2.5 |
| P*tac* | 12.5 | 0.8225 | - | 1 | 2.2 |

The high protein yield (more than 2.5-fold) was detected from the promoters identified upstream of TM0477, TM1016 and TMtRNAser1 genes. Eight other putative promoters upstream of TM0373, TM1067, TMtRNAthr1, TM1429, TM1490, TM1667, TM1780 and TM1271 genes increased ArgC synthesis from 1.6-fold to 2.4-fold. It appeared that the identified promoter upstream of TM0032 is subjected to repression by the endogenous *E. coli* XylR analogue in S30 extracts.

Thus, *E. coli* RNA polymerase provided the ArgC reporter-protein *in vitro* synthesis from the 13 identified *T. maritima* promoter sequences. Moreover, these results indicate that the identified *T. maritima* DNA sequences harbour, indeed, strong promoters, which are active in *E coli* S30 extracts.

**B.3 Example 3: *T. maritima* RNA polymerase α subunit increases the reporter ArgC protein yield *in vitro* from putative T. *maritima* promoters**

Previously it was shown that the addition of *E. coli* RNA polymerase α subunit can increase *in vitro* synthesis of a desired protein expressed from a promoter harbouring a UP-element. Therefore, in this study the effect of the *T. maritima* RNA polymerase α subunit was also tested on a behaviour of the 13 selected *T. maritima* promoters *in vitro*. Indeed, the addition of a purified *T. maritima* RNA polymerase α subunit, in a range from 800 to 2600 nM, stimulated ArgC synthesis from all promoters (Figure 3). Quantitative analysis showed that the reporter-gene encoded protein synthesis is increased by 1.2-fold to 2.7-fold as compared in the absence of an exogenous α subunit (Table 3 ). Protein synthesis was also stimulated from the control strong promoter P*tac* in the presence of the *T. maritima* RNA polymerase α subunit as indicative of the latter's interaction with a heterologous *E. coli* promoter.

Thus, the data presented indicate that transcription from all tested *T. maritima* promoters is subjected to the action of homologous RNA polymerase α subunit. Therefore, one should expect that the strength of these promoters is, at least partially, related with the presence of a AT-rich UP element, which is a target for binding RNA polymerase α subunit. The increase of ArgC protein production *in vitro* by α subunit indicates also that though *T. maritima* strong promoters are occupied by heterologous *E. coli* RNA polymerase from S30 extracts, exogenous *T. maritima* RNA polymerase α subunit can bind to an UP-element of these promoters and provide a higher reporter-gene expression.

**B.4 Example 4: *T. maritima* RNA polymerase α subunit increases protein yield *in vitro* from a native context of the *T. maritima* genome**

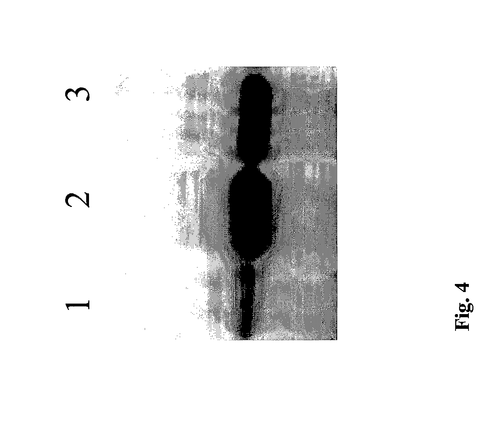

The action of *T. maritima* RNA polymerase α subunit was also tested on a strong P*argG* promoter located upstream of TM1780 and governing transcription of a putative *arg*GHJBCD operon of *T. maritima* by following the ArgG protein synthesis *in vitro*. The P*argG* promoter again mediated a high protein production as observed with the reporter-gene *argC* expression. Moreover, protein synthesis increased nearly 6-fold and 4-fold in the presence respectively, of 500 nM and 1000 nM *T. maritima* RNA polymerase α subunit (Figure 4).

**B.5. Example 5: *T. maritima* and *E. coli* UP elements possess differentconsensus sequences**

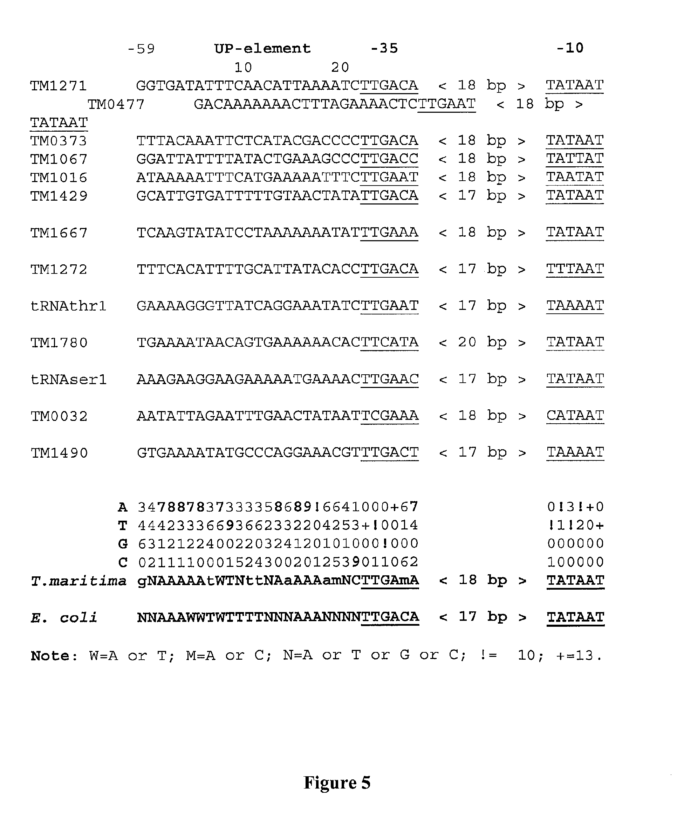

The 13 strong promoters identified in *T. maritima* were aligned that permits to characterize corresponding subregions (Figure 5). The most conserved sequence was found to be -10 site, which is identical to the *E. coli* consensus (TATAAT) recognized by σ70 factor. A high similarity exists also between -35 site of both bacterial promoters though there is not a preference for the 5$^{th}$ symbol of analysed *T. maritima* sequences. In strong promoters of this bacterium, -10 and -35 sites are separated by 18 bp rather, than by 17 bp as in *E. coli*. UP elements of strong promoters from both bacteria also exhibit noticeable similarity as can be judged from two conserved A-tracts (AAA-triplets), which appear to be essential for α subunit contacts and the promoter strength (Gourse *et al.,* 2000). However, UP element of *T. maritima* strong promoters is richer in Adenine and the distal A-tract appears to be longer in *T. maritima* than in *E. coli*. Other possible features are less conserved T-tract in the central part of a full UP element and a preference for Cytosine just before - 35 site in strong promoters of *T. maritima*. It has been supposed that the residue preceding -35 site plays a crucial role in some *E. coli* strong promoters (Estrem *et al.,* 1999). As in *E. coli* the *T. maritima* UP element's AAA-triplets are separated by 11 bp supposing that the same surface of two α subunits determines DNA contacts. However, the presence of longer A-tracts in *T. maritima* allows to assume more dynamism in the capacity of its RNA polymerase to recognise corresponding UP element subsites upstream of -35 consensus.

Thus, the detected features between strong promoter sequences of the two bacteria allow assuming that RNA polymerase-promoter interactions can be somehow different in distant bacteria.

**B.6 Example 6: Identification of strong promoters in other sequenced bacterial genomes**

Next, the algorithm "STRONG_PROMOTERS_SEARCH" was used to identify strong promoters in 46 available bacterial genomes in GenBank (Table 4).

Table 4.

| Number of putative strong promoters in bacterial genomes. | | | | | |
|---|---|---|---|---|---|
| N° | Genome | Length, bp | Number of genes | * | ** |
| 1 | *Deinococcus radiodurans* R1 (AE000513) | 2648638 | 2681 | 5 | 1 |
| 2 | *Pseudomonas aeruginosa* PA01 (AE004091) | 6264403 | 5570 | 15 | 2 |
| 3 | *Mycobacterium tuberculosis* (AL 123456) | 4411529 | 3922 | 7 | 0 |
| 4 | *Caulobacter crescentus* (AE005673) | 4016947 | 3787 | 2 | 0 |
| 5 | *Ralstonia solanacearum* GMI1000 chromosome (AL646052) | 3716413 | 3477 | 7 | 0 |
| 6 | *Xanthomonas campestris pv. campestris str.* ATCC 33913 (AE008922) | 5076188 | 4197 | 2 | 0 |
| 7 | *Xanthomonas axonopodis pv. citri str.* 306 (AE008923) | 5175554 | 4344 | 2 | 0 |
| 8 | *Mesorhizobium loti* NC_002670) | 7036074 | 6693 | 9 | 0 |
| 9 | *Sinorhizobium meliloti* 1021 (AL591688) | 3654135 | 3375 | 8 | 0 |
| 10 | *Mycobacterium leprae* strain TN (AL450380) | 3268203 | 2770 | 8 | 1 |
| 11 | *Agrobacterium tumefaciens strain C58 linear chromosome* (AE007870) | 2074782 | 1825 | 12 | 3 |
| 12 | *Brucella melitensis strain 16M chromosome I* (AE008917) | 2117144 | 2059 | 21 | 4 |
| 13 | *Agrobacterium tumefaciens strain C58 circular chromosome* (AE007869) | 2841581 | 2701 | 20 | 1 |
| 14 | *Treponema pallidum* (AE000520) | 1138011 | 1083 | 4 | 3 |
| 15 | *Chlorobium tepidum TLS (AE006470)* | 2154946 | 2329 | 35 | 13 |
| 16 | *Salmonella typhimurium LT2* (AE006468) | 4857432 | 4608 | 163 | 61 |
| 17 | *Neisseria meningitidis serogroup B* strain MC58 (AE002098) | 2272351 | 2226 | 112 | 45 |
| 18 | *Escherichia coli* O157:H7 (AE005174) | 5528445 | 5478 | 263 | 79 |
| 19 | *Xylella fastidiosa* plasmid pXF51 (AE003851 ) | 51158 | 64 | 4 | 0 |
| 20 | *Vibrio cholerae* chromosome I (AE003852) | 2961149 | 2887 | 93 | 37 |
| 21 | *Yersinia pestis* strain C092 (AL590842) | 4653728 | 4042 | 274 | 61 |
| 22 | *Methanobacterium thermoautotrophicum* delta H (AE000666) | 1751377 | 1900 | 81 | 24 |
| 23 | *Synechocystis PCC6803 (AB001339)* | 3573470 | 1074 | 31 | 6 |
| 24 | *Thermotoga maritima* (AE000512) | 1860725 | 1926 | 63 | 10 |
| 25 | *Aquifex aeolicus* (AE000657) | 1551335 | 1503 | 71 | 37 |
| 26 | *Bacillus halodurans* C-125 (BA000004) | 4202353 | 4125 | 359 | 87 |
| 27 | *Bacillus subtilis* (AL009126) | 4214814 | 4182 | 430 | 111 |
| 28 | *Chlamydia muridarum* (AE002160) | 1069411 | 954 | 86 | 31 |
| 29 | *Mycoplasma pneumoniae* M129 (U00089) | 816394 | 705 | 37 | 14 |

* Number of putative strong promoter sequences in "upstream" regions

** Number of putative strong promoters in "downstream" regions

Table 4. (continued)

| Number of putative strong promoters in bacterial genomes. | | | | | |
|---|---|---|---|---|---|
| N° | Genome | Length, bp | Number of genes | * | ** |
| 30 | *Streptococcus pneumoniae* (AE005672) | 2160837 | 2306 | 365 | 156 |
| 31 | *Helicobacter pylori,* strain J99 (AE001439) | 1643831 | 1495 | 182 | 54 |
| 32 | *Streptococcus pyogenes* strain SF370 serotype M1 (AE004092) | 1852441 | 1731 | 292 | 115 |
| 33 | *Haemophilus influenzae* Rd(L42023) | 1830138 | 1775 | 277 | 94 |
| 34 | *Pasteurella multocida* PM70 (AE004439) | 2257487 | 1996 | 228 | 64 |
| 35 | *Listeria innocua* Clip11262 (AL592022) | 3011208 | 3529 | 426 | 229 |
| 36 | *Chlamydophila pneumoniae* J 138 (BA000008) | 1226565 | 1097 | 162 | 51 |
| 37 | *Thermoanaerobacter tengcongensis* strain MB4T (AE008691 ) | 2689445 | 2632 | 467 | 248 |
| 38 | *Clostridium acetobutylicum* ATCC824 (AE001473) | 3940880 | 3738 | 1685 | 916 |
| 39 | *Mycoplasma genitalium* G37 (L43967) | 580074 | 519 | 83 | 63 |
| 40 | *Staphylococcus aureus strain* N315 (BA000018) | 2814816 | 2638 | 930 | 418 |
| 41 | *Rickettsia prowazekii strain Madrid E* (AJ235269) | 1111523 | 885 | 443 | 252 |
| 42 | *Campylobacter jejuni* (AL111168) | 1641481 | 1684 | 540 | 353 |
| 43 | *Lyme disease spirochete, Borrelia burgdorferi.* (AE000783) | 910724 | 875 | 350 | 292 |
| 44 | *Clostridium perfringens* 13 DNA (BA000016) | 3031430 | 2779 | 1499 | 772 |
| 45 | *Ureaplasma urealyticum* (AF222894) | 751719 | 645 | 328 | 236 |
| 46 | *Buchnera aphidicola str. Sg* (*Schizaphis graminum*) (AE013218) | 641454 | 584 | 339 | 225 |

* Number of putative strong promoter sequences in "upstream" regions

** Number of putative strong promoters in "downstream" regions

The table 4 shows the number of strong promoters putative for each genome. For comparison it includes the number of false "strong promoter-like" regions detected downstream of real promoter regions, namely a search for a 300 bp region after the transcription start site of all genes by the algorithm. The results clearly indicate that the number of strong promoter-like sequences differ dramatically in 300 bp portion located upstream and downstream of the corresponding regions, thereby confirming the validity of at least majority of the identified sequences on a genome scale.

**B.7 Example 7: Number of strong promoters reflects an A+T composition of bacterial genomes**

Since 24 of 29 symbols in all three patterns are *a*'s and *t*'s one can suppose that the percentage of genes with strong promoters depends on the percentage of symbols a and *t* in a given genome. The computational experiments confirm partially this assumption (Table 5).

Table 5.

| Relation between number of putative strong promoters and A+T composition of bacterial genomes | | | | |
|---|---|---|---|---|
| N° | Bacterial genome | at% | strong promoters % | random s.p. % |
| 1 | *Deinococcus radiodurans* R1 (AE000513) | 32,99 | 0,19 | 0 |
| 2 | *Pseudomonas aeruginosa* PA01 (AE004091) | 33,44 | 0,27 | 0 |

Table 5. (continued)

| Relation between number of putative strong promoters and A+T composition of bacterial genomes | | | | |
|---|---|---|---|---|
| N° | Bacterial genome | at% | strong promoters % | random s.p. % |
| 3 | *Mycobacterium tuberculosis* (AL123456) | 34,39 | 0,18 | 0 |
| 4 | *Caulobacter crescents* (AE005673) | 34,40 | 0,05 | 0 |
| 5 | *Ralstonia solanacearum* GM11000 chromosome (AL646052) | 34,51 | 0,20 | 0 |
| 6 | *Xanthomonas campestris pv. campestris* str. ATCC 33913 (AE008922) | 35,64 | 0,05 | 0 |
| 7 | *Xanthomonas axonopodis pv. citri str.* 306 (AE008923) | 36,02 | 0,05 | 0 |
| 8 | *Mesorhizobium loti* NC_002670) | 39,09 | 0,13 | 0 |
| 9 | *Sinorhizobium meliloti* 1021 (AL591688) | 39,66 | 0,24 | 0 |
| 10 | *Mycobacterium leprae strain* TN (AL450380) | 42,20 | 0,29 | 0 |
| 11 | *Agrobacterium tumefaciens strain* C58 linear chromosome (AE007870) | 42,68 | 0,66 | 0 |
| 12 | *Brucella melitensis* strain 16M chromosome I (AE008917) | 42,84 | 1,02 | 0 |
| 13 | *Agrobacterium tumefaciens* strain C58 circular chromosome (AE007869) | 43,20 | 0,74 | 0 |
| 14 | *Treponema pallidum* (AE000520) | 47,01 | 0,37 | 0 |
| 15 | *Chlorobium tepidum* TLS (AE006470) | 47,50 | 1,50 | 0,303 |
| 16 | *Salmonella typhimurium* LT2 (AE006468) | 47,78 | 3,54 | 0,306 |
| 17 | *Neisseria meningitidis* serogroup B strain MC58 (AE002098) | 48,47 | 5,03 | 0,33 |
| 18 | *Escherichia coli* O157:H7 (AE005174) | 49,50 | 4,80 | 0,48 |
| 19 | *Xylella fastidiosa* plasmid pXF51 (AE003851) | 51,43 | 6,25 | 0,84 |
| 20 | *Vibrio cholerae* chromosome (AE003852) | 52,30 | 3,22 | 1 |
| 21 | *Yersinia pestis* strain CO92 (AL590842) | 52,36 | 6,78 | 1,08 |
| 22 | *Methanobacterium thermoautotrophicum* delta H (AE000666) | 53,11 | 4,26 | 1,28 |
| 23 | *Synechocystis* PCC6803 (AB001339) | 53,71 | 2,89 | 1,7 |
| 24 | *Thermotoga maritima* (AE000512) | 53,75 | 3,27 | 1,75 |
| 25 | *Aquifex aeolicus* (AE000657) | 57,73 | 4,72 | 4,05 |
| 26 | *Bacillus halodurans* C-125 (BA000004) | 58,65 | 8,70 | 4,75 |
| 27 | *Bacillus subtilis* (AL009126) | 59,30 | 10,28 | 5,7 |
| 28 | *Chlamydia muridarum* (AE002160) | 59,69 | 9,01 | 6,6 |
| 29 | *Mycoplasma pneumoniae* M129 (U00089) | 59,99 | 5,25 | 7,35 |
| 30 | *Streptococcus pneumoniae* (AE005672) | 60,30 | 15,83 | 7,7 |
| 31 | *Helicobacterpylori,* strain J99 (AE001439) | 60,81 | 12,17 | 8,35 |
| 32 | *Streptococcus pyogenes* strain SF370 serotype M1 (AE004092) | 61,49 | 16,87 | 9,5 |
| 33 | *Haemophilus influenzae* Rd (L42023) | 61,85 | 15,61 | 9,9 |

Table 5. (continued)

| Relation between number of putative strong promoters and A+T composition of bacterial genomes | | | | |
|---|---|---|---|---|
| **N°** | **Bacterial genome** | **at%** | **strong promoters %** | **random s.p. %** |
| 34 | *Pasteurella multocida* PM70 (AE004439) | 62,31 | 11,42 | 10,9 |
| 35 | *Listeria* innocua Clip11262 (AL592022) | 62,56 | 12,07 | 11,5 |
| 36 | *Chlamydophila pneumoniae* J138 (BA000008) | 62,80 | 14,77 | 12,5 |
| 37 | *Thermoanaerobacter tengcongensis strain MB4T* (AE008691) | 64,11 | 17,74 | 14,8 |
| 38 | *Clostridium acetobutylicum* ATCC824 (AE001473) | 69,07 | 45,08 | 32,9 |
| 39 | *Mycoplasma genitalium* G37 (L43967) | 69,50 | 15,99 | 35 |
| 40 | *Staphylococcus aureus* strain N315 (BA000018) | 69,71 | 35,25 | 35,5 |
| 41 | *Rickettsia prowazekii* strain Madrid E (AJ235269) | 71,00 | 50,06 | 40,2 |
| 42 | *Campylobacter jejuni* (AL111168) | 71,36 | 32,07 | 41,8 |
| 43 | *Lyme disease spirochete, Borrelia burgdorferi*. (AE000783) | 71,40 | 40,00 | 42 |
| 44 | *Clostridium perfringens* 13 DNA (BA000016) | 71,43 | 53,94 | 42,1 |
| 45 | *Ureaplasma urealyticum* (AF222894) | 76,05 | 50,85 | 65,35 |
| 46 | *Buchnera aphidicola str. Sg (Schizaphis graminum)* (AE013218) | 78,36 | 58,05 | 74,5 |

The third column "at%" shows the percentage of symbols a and *t* into genomes, the next column "strong promoters %" shows the percentage of genes with strong promoters among all genes of genomes. The following score parameters where used: *scup=13.0, sc35= 5.5, sc10 = 5.0.* The last column shows the percentage of genes with strong promoters among random upstream regions which where generated with the same percentage of a's and *t*'s as in the corresponding "real" genomes.

This table shows that genomes with rather small percentage a's and *t*'s (less than 50%) have much more genes transcribed from strong promoters as compared from "random genomes" with a similar percentage a's and t's. When percentage a's and *t*'s grows from 50% to 65% the difference between the percentage of strong promoters into real and random genomes decreases but still is meaningful enough. However, this difference disappears when the percentage *a*'s and *t*'s exceeds 65%. There are some exceptions. For example, three tested mycoplasmial genomes (data are shown for a single representative) have relatively low percentage of genes transcribed from strong promoter.

Thus, the developed algorithm permits to identify strong putative promoters in bacterial genomes. The algorithm is based on the identification of promoters containing an UP-element and conservative -10 and -35 sites separated by 17 bp. The putative highly expressed bacterial genes can be clustered into several groups, which include essential for cellular growth genes for translation, protein transport and protein folding as well as " non-essential " or non-yet identified ones. It appears that functions of "non-essential" genes are related with providing large quantities of encoded proteins required to adapt to various extra-cellular environmental conditions.

The strength of putative promoters has been proven experimentally for 13 putative promoter sequences of a hyperthermophilic bacterium *T. maritima* using a reporter-gene expression from a linear DNA template in a coupled transcription-translation system. Though such an evaluation may diminish a real promoter strength because of gene expression by a heterologous RNA polymerase holoenzyme, but the proposed approach avoids time-consuming steps for DNA cloning in cells. The method can be especially useful for simultaneous and rapid characterization of numerous putative promoters in bacterial genomes, including pathogens. All *T. maritima* promoters were found to mediate high protein synthesis *in vitro*. Moreover, the addition of the purified α subunit of *T. maritima* or *E. coli* RNA polymerase increases the protein yield from all tested promoters, thereby proving the essential role of RNA polymerase α subunit/UP element interactions for determining the promoter strength. Indeed, this subunit is able to bind the promoter sequences as shown by the protein array method for several cases.

The data presented show that the behaviour of some strong promoters depends on interactions with heterologous transcription regulatory proteins in *E. coli* S30 extracts that appears to prohibit binding α subunit of *T. maritima* RNA polymerase to DNA targets and, thereby decrease protein expression.
The identified strong promoters from various bacterial sources can be used both for the construction of new expression vectors and protein overproduction in cellular and cell-free systems.
Furthermore, the identified strong promoters in pathogenic bacteria, for example in *Mycobacterium tuberculosis, Mycobacterium leprae, Pseudomonas aeruginosa, Brucella melitensis, Neisseria meningitis, Salmonella typhimurium, Escherichia coli, Vibrio cholerae, Yersinia pestis, Streptococcus pneumoniae, Streptococcus pyogenes, Haemophilus influenzae* and *Helicobacter pylori* are also attractive as potential targets for development of new antibacterial therapy approaches.

## REFERENCES

Aiyar, S. E., Gourse, R. L. & Ross, W. (1998). Upstream A-tracts increase bacterial promoter activity through interactions with the RNA polymerase alpha subunit. *Proc. Natl; Acad. Sci. USA* **95,** 14652-14657.

Aiyar, S. E., Gaal, T. & Gourse, R. L. (2002). rRNA promoter activity in the fast-growing bacterium *Vibrio natrigens. J. Bacter.* **184,** 1349-1358.

Altschul S., Gish W., Miller E., Myers E., and Lipman J. (1990). Basic local alignment search tool. J. Mol. Biol. 215, 403-410.

Chen, G., Dubrawsky, I., Mendez, P., Georgiou, G. & Iverson, B. L. (1999). *In vitro* scanning saturation mutagenesis of all the specificity determining residues in an antibody binding site. *Protein Eng.* **12,** 349-356.

Dimova D., Weigel P., Takahashi M., Marc F., Van Duyne G. D. & .Sakanyan, V. (2000). Thermostability, oligomerisation and DNA-binding properties of the regulatory protein ArgR from the hyperthermophilic bacterium *Thermotoga neapolitana. Mol. Gen. Genet.* **263,** 119-130.

Estrem, S. T., Gaal, T., Ross, W. & Gourse, R. L. (1998). Identification of an UP element consensus sequence for bacterial promoters. *Proc. Natl. Acad. Sci. USA* **95,** 9761-9766.

Estrem, S. T., Ross, W., Gaal, T., Chen, Z. W. S., Niu, W., Ebright, R. H. & Gourse, R. L. (1999). Bacterial promoter architecture: subsite structure of UP elements and interactions with the C-terminal domain of the RNA polymerase $\alpha$ subunit. *Genes & Dev.* **13,** 2134-2147.

Fredrick, K., Caramori, T., Chen, Y. F., Galizzi, A.& Helmann, J. D. (1995). Promoter architecture in the flagellar regulon of *Bacillus subtilis*: high-level expression of flagellin by the sigma D RNA polymerase requires an upstream promoter element. *Proc. Natl. Acad. Sci. USA* **92,** 2582-2586.

Gourse, R. L., Ross, W. & Gaal, T. (2000). Ups and downs in bacterial transcription initiation: the role of the alpha subunit of RNA polymerase in promoter recognition. *Mol. Microbiol.* **37,** 687-695.

Graves, M. C. & Rabinowitz, J. C. (1986). *In vivo* and *in vitro* transcription of the *Clostridium pasterianum* ferredoxin gene. Evidence for "extended" promoter elements in gram-positive organisms. *J. Biol. Chem.* **261,** 11409-11415.

Ho, N. S., Hunt, D. H., Horton, M. R., Pullen K. J. & Pease R., L. (1989). Site directed mutagenesis by overlap extension using the polymerase chain reaction. *Gene* **77,** 51-59.

Kigawa, T., Yabuki, T., Yoshida, Y., Tsutsui, M., Ito, Y., Shibata, T. & Yokoyama, S. (1999). Cell-free production and stable-isotope labeling of milligram quantities of proteins. *FEBS Letters* **442,** 15-19.

Kim, D.-M. & Swartz, J. R. (1999). Prolonging cell-free protein synthesis with a novel ATP regeneration system. *Biotech. & Bioengin.* **66,** 180-188.

Kimura, M. & Ishihama, A. (1996). Subunit assembly *in vivo* of *Escherichia coli* RNA polymerase: role of the amino-terminal assembly domain of alpha subunit. *Genes Cells* **1,** 517-28.

Lesley, S.S., Borw, M.A. & Burgess, R.R. (1991). Use of *in vitro* protein synthesis from polymerase chain reaction-generated templates to study interaction of *Escherichia coli* transcription factors with core RNA polymerase and for epitope mapping of monoclonal antibodies. *J. Biol. Chem.* 266, 2632-2638.

Mattheakis, L. C., Dias, J. M. & Dower, W. J. (1996). Cell-free synthesis of peptide libraries displaied on polysomes. *Meth. Enzymol.* **267,** 195-207.

Nelson, K. E. *et al*. (1999). Evidence for lateral gene transfer between Archaea and Bacteria from genome sequence of *Thermotoga maritima. Nature* **399,** 323-329.

Pelham, H.R. & Jackson, R.J. (1976). An efficient mRNA-dependent translation system from reticulocyte lysates. *Eur. J. Biochem.* **67,** 247-256.

Roberts, B.E. & Paterson, B.M. (1973). Efficient translation of tobacco mosaic virus RNA and rabbit globin 9S RNA in a cell-free system from commercial wheat germ. *Proc. Natl. Acad. Sci.* USA **70,** 2330-2334.

Ross, W., Gosink, K. K., Salomon, J., Igarashi, K., Zou, C., Ishihama, A., Severinov, K. & Gourse, R. L. (1993). A third recognition element in bacterial promoters: DNA binding by the $\alpha$ subunit of RNA polymerase. *Science* **262,** 1407-1413.

Ross, W., Ernst, A. & Gourse, R. L. (2001). Fine structure of *E. coli* RNA polymerase-promoter interactions: $\alpha$ subunit binding to the UP element minor groove. *Genes & Dev.* **15,** 491-506.

Sambrook *et al.* (2001). Molecular Cloning: A laboratory Manual, 3rd Ed., Cold Spring Harbor, laboratory press, Cold Spring Harbor, New York.

Sakanyan, V. A., Hovsepyan, A. S., Mett, I. L., Kochikyan, A. V. & Petrosyan, P. K. (1990). Molecular cloning and structural-functional analysis of arginine biosynthesis genes of the thermophilic bacterium *Bacillus stearothermophilus. Genetika* (USSR) **26,** 1915-1925.

Sakanyan, V., Charlier, D., Legrain, C., Kochikyan, A., Mett, I., Piérard, A. & Glansdorff, N. (1993). Primary structure, partial purification and regulation of key enzymes of the acetyl cycle of aginine biosynthesis in *Bacillus stearothermophilus*: dual function of ornithine acetyltransferase. *J. Gen. Microbiol.* **139,** 393-402.

Savchenko A., Weigel P., Dimova D., Lecocq M. & Sakanyan V. (1998). The *Bacillus stearothermophilus argCJBD* operon harbours a strong promoter as evaluated in *Escherichia coli* cells. *Gene* 212, 167-177.Studier, F. W., Rosenberg, A. H., Dunn, J. J. & Dubendorff, J. W. (1990). Use of T7 RNA polymerase to direct expression of cloned genes. *Methods Enzymol.* 185, 60-89.

Thieffry, D., Salgado, H., Huerta, A. M. & Collado-Vides, J. (1998). Prediction of transcriptional regulatory sites in the complete genome sequence of *Escherichia coli* K-12. *Bioinformatics* **14,** 391-400.

Thorson, J.S., Cornish, V.W., Barrett, J.E., Cload, S.T., Yano, T. & Schultz, P.G. (1998). A biosynthetic approach for the incorporation of unnatural amino acids into proteins. In: *Methods Mol. Biol.* vol. 77, Protein Synthesis: methods and protocols. Ed. R. Martin, Humana Press Inc., Totowa, NJ, p. 43-73.

Van Essen, A.J., Kneppers, A.L., van der Hout, A.H., Scheffer, H., Ginjaar, I.B., ten Kate, L.P., van Ommen, G.J., Buys, C.H. & Bakker, E. (1997). The clinical and molecular genetic approach to Duchenne and Becker muscular dystrophy: an updated protocol. *J. Meth. Genet.* 34, 805-812.

Zubay, G. (1973). *In vitro* synthesis of protein in microbial systems. *Ann. Rev. Genet.* 7, 267-287.

## Claims

**1.** A method for the identification of a nucleic acid sequence carrying a putative bacterial strong promoter, said method comprising:

a. selecting among the sequences of a nucleic acid database, a putative promoter sequence of at least 50 nucleotides, preferably around 60-70 nucleotides, said putative promoter sequence being located upstream the initiation codon of an Open Reading Frame or a sequence corresponding to tRNA or rRNA, in a region which does not extend further than 500 nucleotides, preferably 300 nucleotides from said initiation codon, said putative promoter sequence comprising an UP element, said UP element consisting of either

- the following consensus pattern: AAAWWTWTTTTNNNAAA (SEQ ID NO:1), wherein "W" stands for any of the symbols "A" or "T" and "N" stands for any of the four symbols "A", "T", "G" or "C"; or,
- a nucleotide sequence of the same length of SEQ ID NO:1 which can be aligned with SEQ ID NO:1 and having a score similarity *sUP* which is equal or superior to a minimum score similarity determined by the parameter *scUP*,

b. selecting among the sequences selected in step a., the sequences comprising a -35 site located from 0 to 5 nucleotides downstream the AT-rich UP element, said -35 site consisting of either

- the following consensus pattern TCTTGACAT (SEQ ID NO:2), or
- a nucleotide sequence of the same length of SEQ ID NO:2 which can be aligned with SEQ ID NO:2 and having a score similarity s35 which is equal or superior to a minimum score similarity parameter *sc35*; and

c. identifying among the sequences selected in step b., a sequence comprising a -10 site, downstream the -35 site, preferably at a distance of 14 to 20 nucleotides, preferably from 15 to 19, better from 16 to 18, and optimally 17 nucleotides from the -35 site, said -10 site consisting of either

- the following consensus pattern TATAAT (SEQ ID NO:3), or
- a nucleotide sequence of the same length of SEQ ID NO:3 which can be aligned with SEQ ID NO:3 and having a score similarity *s10* which is equal or superior to a minimum score similarity parameter *sc10*;

wherein *sUP*, *s35* and *s10* correspond to the sum of each coincidence rates of symbols in the corresponding alignments: the identity rate being equal to 1 and the non-identity rate being equal to 0.5 or 0 and determined for each pair compared of symbols as follows:
0.5 for pairs "A" to "T" or "T" to "A" and
0 for other possible pairs.

**2.** The method according to Claim 1, wherein *scUP* is at least equal to 11, *sc35* is at least equal to 5, and *sc10* is at least equal to 4.

**3.** The method according to Claim 1, wherein a normalised score tot_sc is attributed to each identified sequence according to the following equation:

$$tot_sc = 0.30*[1-(17- sUP)/20] + 0.25*[1-(9- sc35)/10] + 0.25*[1-(6- s10)^2/10] + 0.2*nsc_dist,$$

wherein nsc_dist is defined according to the following table:

| Distance between -35 site and -10 site in nucleotides | 17 | 16, 18 | 15, 19 | 14, 20 | Other |
|---|---|---|---|---|---|
| *nsc_dist* | 1 | 0.95 | 0.85 | 0.7 | 0.2 |

and the method further comprises the step of selecting the sequences having a normalised score tot_sc superior to 0.85.

**4.** The method according to any of Claims 1 to 3, wherein said bacterial nucleic acid database comprise genomic sequence from bacteria which is used in industry and whose genome comprises a percentage of adenine and thymine inferior to 65%.

**5.** The method according to any of Claims 1 to 3, wherein said bacterial nucleic acid database comprise genomic sequence from one bacterial specie selected from the group consisting of *Thermotoga maritima, Mycobacterium tuberculosis, Mycobacterium leprae, Pseudomonas aeruginosa, Brucella melitensis, Neisseria meningitis, Salmonella typhimurium, Escherichia coli, Vibrio cholerae, Yersinia pestis, Streptococcus pneumoniae, Streptococcus pyogenes, Haemophilus influenzae* and *Helicobacter pylori*.

**6.** The method according to Claim 5, wherein said bacterial nucleic acid database comprises *T. maritima* genomic sequences.

**7.** A computer program comprising computer program code means for instructing a computer to perform the method of any of Claims 1 to 6.

**8.** A computer readable storage medium having stored therein a computer program according to Claim 7.

**9.** A method for the isolation of a nucleic acid having strong bacterial promoter activity, wherein said method further comprises the steps of:

a. isolating a nucleic acid having a putative strong bacterial promoter, said nucleic acid sequence being identified according to the method of Claim 1,
b. determining promoter activity of the isolated nucleic acid as compared to a control bacterial strong promoter, such as the ptac promoter,

wherein a higher promoter activity than the promoter activity of the control strong promoter indicates that said isolated nucleic acid has a strong bacterial promoter activity.

**10.** An isolated nucleic acid having a strong bacterial promoter activity, **characterized in that** it is obtainable by the method according to Claim 9 and **in that** it consists of

a. a nucleic acid sequence selected among the group consisting of SEQ ID NOs 4-16;
b. a modified nucleic acid sequence having at least 70%, preferably at least 80%, and better at least 90% identity when aligned with one of SEQ ID NOs 4-16,
c. a modified nucleic acid sequence which hybridizes under stringent conditions with one of the sequences of SEQ ID NOs 4-16, or,
d. a nucleic acid sequence comprising the following consensus pattern: GNAAAAATWTNTTNAAAAAAM-NCTTGAMA$(N)_{18}$TATAAT (SEQ ID NO:21), wherein "W" stands for any of the symbols "A" or "T", "N" stands for any of the four symbols "A", "T", "G" or "C", and "M" stands for "A" or "C",

wherein said modified nucleic acid is between 50 and 300 nucleotides long, preferably between 50 and 100 nucleotides long, and retains substantially the same promoter activity as the non-modified sequence to which it can be aligned.

**11.** An expression cassette suitable for inserting into, a sequence encoding a protein of interest and for synthesizing said protein into a host cell or in a cell-free system, comprising a nucleic acid having strong bacterial promoter activity according to Claim 10.

**12.** The expression cassette according to Claim 11, **characterized in that** it is a plasmid, a cosmid or a phagemid for *in vivo* protein synthesis.

**13.** The expression cassette according to Claim 11 or 12, **characterized in that** it further comprises an Open Reading Frame encoding α subunit of a RNA polymerase under the control of a promoter appropriate for expression in said host cell.

**14.** A DNA template for RNA or polypeptide synthesis, comprising a nucleic acid having strong bacterial promoter activity according to Claim 10 located upstream an Open Reading Frame encoding a protein of interest.

**15.** The DNA template according to Claim 14, wherein said DNA template is a linear DNA template further comprising an additional DNA fragment, which is at least 3 bp long, preferably longer than 100 bp and more preferably longer than 200 bp, located immediately downstream the stop codon of said Open Reading Frame.

**16.** A use of the DNA template according to Claim 14 or Claim 15 in a method for RNA or polypeptide synthesis from a DNA template comprising the steps of

a. providing a cellular or cell-free system enabling RNA or polypeptide synthesis from the DNA template according to Claim 14 or Claim 15;
b. recovering said synthesized RNA or polypeptide.

**17.** The use according to Claim 16, wherein the concentration of α subunit of RNA polymerase, but not of other subunits, is increased in said cellular or cell-free system, comparing to its natural concentration.

**18.** The use according to Claim 16 or 17, wherein said system enabling RNA or polypeptide synthesis is a cellular system, which includes culturing recombinant host cells comprising, incorporated therein, the DNA template according to Claim 14 or Claim 15.

**19.** The use according to Claim 18, wherein said host cell is a bacteria, preferably an *Escherichia coli* cell.

**20.** The use according to Claim 18, wherein the cellular concentration of α subunit of RNA polymerase is increased by overexpressing in the host cell, a gene encoding an α subunit of RNA polymerase.

**21.** The use according to Claim 20, wherein said concentration is increased by induction of the expression of an additional copy of a gene encoding α subunit of RNA polymerase while expression of other subunits remains unchanged.

**22.** The use according to Claim 16 or 17, wherein said system enabling RNA or polypeptide synthesis from a DNA template is a cell-free system comprising a bacterial cell-free extract.

**23.** The use according to Claim 22, wherein said cell-free system further comprises purified thermostable RNA polymerase holoenzyme.

**24.** The use according to Claim 23, wherein said thermostable RNA polymerase holoenzyme is from *Thermus thermophilus.*

**25.** The use according to any of Claims 22 to 24, wherein the concentration of α subunit of RNA polymerase is increased by adding purified α subunit of RNA polymerase to the bacterial cell-free extract.

**26.** The use according to Claim 25, wherein said purified α subunit is added to a final concentration comprised between 15 μg/ml and 200 μg/ml.

**27.** A use of an isolated nucleic acid having strong bacterial promoter activity according to Claim 10, for the screening of antibacterial agents which bind to said isolated nucleic acid having strong bacterial promoter activity.

**TM1271 (115 bp) (SEQ ID NO: 4)**

CGGTTTGTCTTTGAGACGAATTTAACAGTTTTCTTCTGTTCTTAGCG***GGTGATATTTCAACA***
***TTAAAATC*****TTGACATTCTACCATGTCAAGGTGTATAAT**GCAAAATGTGAAAAT

**TM0373 (105 bp) (SEQ ID NO: 5)**

ATATTCGATTTCCCTCATATTTAGGTGCATGTATGTT***TTTACAAATTCTCATACGACCCC*****TT**
**GACATCCCATTCTGTGCCTCACTATAAT**TTTTCATGGATGAGA

**TM1067 (142 bp) (SEQ ID NO: 6)**

GGGGTTGTAAGCAAAAGGAAAACTAATATAATCAATAATAATCAACCATATTTATCTCTTAT
AGTTCGATATTA***GGATTATTTTATACTGAAAGCCC*****TTGACCTTGTTGTATGTTTGTTGATAT**
**TAT**ATTGATAACTTCAAG

**TM1429 (131 bp) (SEQ ID NO: 7)**

GAAAGAAGACGTGGAAAGATTTAAAATTTAACAGAAAATAACAACTTCCACATAAGATGAAA
CT***GCATTGTGATTTTTGTAACTATA*****TTGACATAAAACAAAAGGTTTGTTATAAT**TCACATCG
AGGCATA

**TM1667 (123 bp) (SEQ ID NO: 8)**

CCTCTCTGAGCTCTTCTATTCTTTTTGTGATCTCCATTGAAAACACCTCCCAGAT***TCAAGTA***
***TATCCTAAAAAAATAT*****TTGAAATGATACCCCAAGATTTTATATAATT**GATTGATAGAAAAA

**TM1272 (111 bp) (SEQ ID NO: 9)**

CTCATAAAATCCACCTCCCGGGTTAAAAATTGTTAAATATAGAT***TTTCACATTTTGCATTAT***
***ACACC*****TTGACATGGTAGAATGTCAAGATTTTAAT**GTTGAAATATCACCC

**Figure 1A**

**TM1271 (115 bp) (SEQ ID NO: 4)**

CGGTTTGTCTTTGAGACGAATTTAACAGTTTTCTTCTGTTCTTAGCG***GGTGATATTTCAACA***
***TTAAAATC*****TTGACATTCTACCATGTCAAGGTGTATAAT**GCAAAATGTGAAAAT

**TM0373 (105 bp) (SEQ ID NO: 5)**

ATATTCGATTTCCCTCATATTTAGGTGCATGTATGTT***TTTACAAATTCTCATACGACCCC*****TT**
**GACATCCCATTCTGTGCCTCACTATAAT**TTTTCATGGATGAGA

**TM1067 (142 bp) (SEQ ID NO: 6)**

GGGGTTGTAAGCAAAAGGAAAACTAATATAATCAATAATAATCAACCATATTTATCTCTTAT
AGTTCGATATTA***GGATTATTTTATACTGAAAGCCC*****TTGACCTTGTTGTATGTTTGTTGATAT**
**TAT**ATTGATAACTTCAAG

**TM1429 (131 bp) (SEQ ID NO: 7)**

GAAAGAAGACGTGGAAAGATTTAAAATTTAACAGAAAATAACAACTTCCACATAAGATGAAA
CT***GCATTGTGATTTTTGTAACTATA*****TTGACATAAAACAAAAGGTTTGTTATAAT**TCACATCG
AGGCATA

**TM1667 (123 bp) (SEQ ID NO: 8)**

CCTCTCTGAGCTCTTCTATTCTTTTTGTGATCTCCATTGAAAACACCTCCCAGAT***TCAAGTA***
***TATCCTAAAAAAATAT*****TTGAAATGATACCCCAAGATTTTATATAATT**GATTGATAGAAAAA

**TM1272 (111 bp) (SEQ ID NO: 9)**

CTCATAAAATCCACCTCCCGGGTTAAAAATTGTTAAATATAGAT***TTTCACATTTTGCATTAT***
***ACACC*****TTGACATGGTAGAATGTCAAGATTTTAAT**GTTGAAATATCACCC

**Figure 1B**

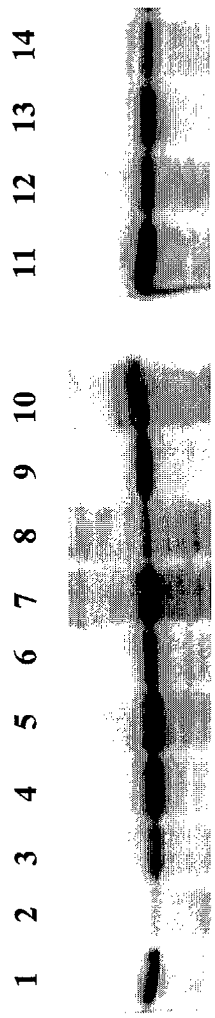


Fig. 2

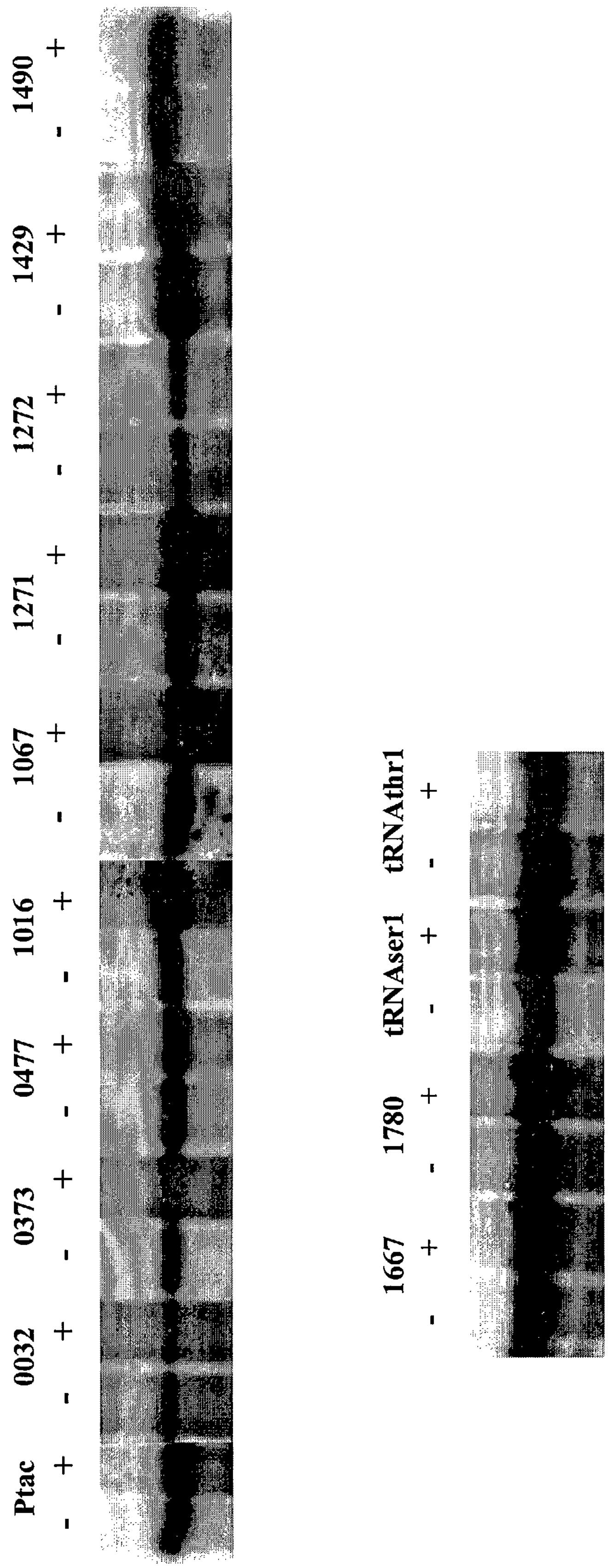
Ptac
0032
0373
0477
1016
1067
1271
1272
1429
1490
1667
1780
tRNAser1
tRNAthr1

Fig. 3

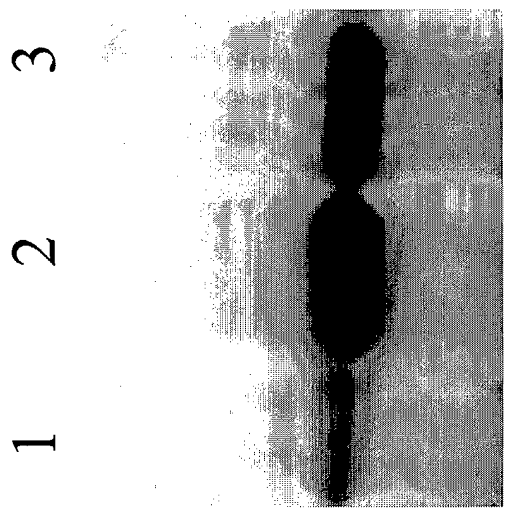


Fig. 4

```
            -59     UP-element      -35                  -10
                       10        20
TM1271      GGTGATATTTCAACATTAAAATCTTGACA   < 18 bp >   TATAAT
TM0477      GACAAAAAAACTTTAGAAAACTCTTGAAT   < 18 bp >   TATAAT
TM0373      TTTACAAATTCTCATACGACCCCTTGACA   < 18 bp >   TATAAT
TM1067      GGATTATTTTATACTGAAAGCCCTTGACC   < 18 bp >   TATTAT
TM1016      ATAAAAATTTCATGAAAAATTTCTTGAAT   < 18 bp >   TAATAT
TM1429      GCATTGTGATTTTTGTAACTATATTGACA   < 17 bp >   TATAAT

TM1667      TCAAGTATATCCTAAAAAAATATTTGAAA   < 18 bp >   TATAAT

TM1272      TTTCACATTTTGCATTATACACCTTGACA   < 17 bp >   TTTAAT

tRNAthr1    GAAAAGGGTTATCAGGAAATATCTTGAAT   < 17 bp >   TAAAAT

TM1780      TGAAAATAACAGTGAAAAAACACTTCATA   < 20 bp >   TATAAT

tRNAser1    AAAGAAGGAAGAAAAATGAAAACTTGAAC   < 17 bp >   TATAAT

TM0032      AATATTAGAATTTGAACTATAATTCGAAA   < 18 bp >   CATAAT

TM1490      GTGAAAATATGCCCAGGAAACGTTTGACT   < 17 bp >   TAAAAT


          A 34788783733335868 9!6641000+67              0!3!+0
          T 44423336693662332204253+!0014               !1!20+
          G 63121224002203241201010 00!000              000000
          C 02111100015243002012539011062               100000
T.maritima  gNAAAAAtWTNttNAaAAAamNCTTGAmA   < 18 bp >   TATAAT

E. coli     NNAAAWWTWTTTTNNNAAANNNNTTGACA   < 17 bp >   TATAAT

Note: W=A or T; M=A or C; N=A or T or G or C; != 10; +=13.
```

**Figure 5**

```
LOCUS       AE000512  1860725 bp    DNA   circular  BCT
DEFINITION  Thermotoga maritima complete genome.

 Total number of  genes on the 'direct' strand: 1056

     UP-ELEMENTS for genes on the 'direct' strand
=================================================================
     gene            11547..12308 /gene="TM0013"
          /product="conserved hypothetical protein"

up_element: 11384  total score: 0,8475

Scores: up-element  -35 pos.               -10 pos.
          15           6,5   <---- 14 ---->   5
ACAATTTTTATCTGATAtttTTTTCACATtcaccatagtcgatTATAAC
<---- 97 bp ---->aatctggaggtgacaatATG
-----------------------------------------------------------------
     gene            12318..13154 /gene="TM0014"
          /product="methyl-accepting chemotaxis protein, putative"
^^^^^^^^^^^^^^^^^^^^^ End of operon (2 genes)^^^^^^^^^^^^^^^^^^^^^
=================================================================
     gene            13245..13823 /gene="TM0015"
          /product="pyruvate ferredoxin oxidoreductase, gamma

up_element: 13154  total score: 0,8375

Scores: up-element  -35 pos.               -10 pos.
          14           5,5   <---- 15 ---->   5
ATTTATTGTTTAAGTATaccaTTATGAAATgcattatgtatctgaTACAAT
<---- 23 bp ---->cgaggaggtgcaatcggATG
-----------------------------------------------------------------
     gene            13830..14135 /gene="TM0016"
          /product="pyruvate ferredoxin oxidoreductase, delta
     gene            14132..15316 /gene="TM0017"
          /product="pyruvate ferredoxin oxidoreductase, alpha
     gene            15331..16305 /gene="TM0018"
          /product="pyruvate ferredoxin oxidoreductase, beta
     gene            16347..17117 /gene="TM0019"
          /product="oxidoreductase, short chain
     gene            17114..17497 /gene="TM0020"
          /product="conserved hypothetical protein"
     gene            17494..17802 /gene="TM0021"
          /product="conserved hypothetical protein"
^^^^^^^^^^^^^^^^^^^^^ End of operon (7 genes)^^^^^^^^^^^^^^^^^^^^^
=================================================================
```

**Figure 6A**

```
     gene            32828..34015 /gene="TM0033"
          /product="hypothetical protein"

up_element: 32689  total score: 0,8425

Scores: up-element  -35 pos.               -10 pos.
         13            5,5    <------ 18 ------>   5
AGAATGTGTGTAACAAAaaatCCATCGAAATcataagttattgactccaTGTAAT
<---- 67 bp ---->taatcacgagaagaggaATG
=================================================================
     gene            47182..47646 /gene="TM0050"
          /product="iron(II) transport protein A"


up_element: 47081  total score: 0,8675

Scores: up-element  -35 pos.               -10 pos.
         14             5,5   <----- 17 ----->   5
GACAATATATTAGAAATgtataTATTGAAAGcatccatgtgatgatgaTACAAT
<---- 30 bp ---->aaaaaggggcagagccGTG
-----------------------------------------------------------------
     gene            47615..49624 /gene="TM0051"
          /product="iron(II) transport protein B"
     gene            49621..49953 /gene="TM0052"
          /product="hypothetical protein"
     gene            49950..51044 /gene="TM0053"
          /product="esterase, putative"
     gene            51041..51631 /gene="TM0054"
          /product="hypothetical protein"
^^^^^^^^^^^^^^^^^^^ End of operon (5 genes)^^^^^^^^^^^^^^^^^^^^

     gene            complement(1831576..1831755) /gene="TM1850"
          /product="hypothetical protein"

Position of up_element:  1831867  total score: 0,855

Scores:  up-element  -35 pos.            -10 pos.
         13,5           6,5   <---- 15 ---->   5
Complement:
AAACGATTCTTCTAAAAtgtgtTCTTGATTTgtatcactgttatgtTATAAA
<---- 43 bp ---->aaaaaggaggtgaaactATG
Direct:
TTTATAacataacagtgatacAAATCAAGAacacaTTTTAGAAGAATCGTTT
```

**Figure 6B**

| Downstream gene | Characteristics of promoter | Score | SEQ ID NO: |
|---|---|---|---|
| TM0013 conserved hypothetical protein Operon: 2 genes | ACAATTTTTATCTGATATTTTCACATtca ccatagtcgatTATAAC | 0,8475 | 51 |
| TM0015 pyruvate ferredoxin oxidoreductase Operon: 7 genes | ATTTATTGTTTAAGTATTTATGAAATgca ttatgtatctgaTACAAT | 0,8375 | 52 |
| TM0033 hypothetical protein | AGAATGTGTGTAACAAACCATCGAAAT cataagttattgactccaTGTAAT | 0,8425 | 53 |
| TM0050 iron(II) transport protein A Operon: 5 genes | GACAATATATTAGAAATTATTGAAAGca tccatgtgatgatgaTACAAT | 0,8675 | 54 |
| TM0110 XylR-related trans-criptional regulator Operon: 6 genes | ACCTTGATTTTAAATTATCCTGCATataa ttaatgtgaaCATAAT | 0,805 | 55 |
| TM0122 ferric uptake regulation protein Operon: 6 genes | ACAATGTTTGAATGATACTTGAAATcag cgactgtgtgtagTACAAT | 0,8425 | 56 |
| TM0277 | AGATTTATGTTAACTAAACTATAAGATa attctttgttgacagaTATGAT | 0,875 | 57 |
| TM0280 hypothetical protein Operon: 8 genes | CAATATTTGTCCAGAAAACTTGATTTaa caaaaatggacaatgTAGAAT | 0,88 | 58 |
| TM0300 oligopeptide ABC transporter | AAATGTGATATGAAAAATATGGAACGA TaagttatcatatttcttTTTAAT | 0,8675 | 59 |
| TM0339 hypothetical protein Operon: 3 genes | AGAAAAATTTTTTTGGAACTTGACAAaa tatttggtaatattcTAAAAT | 0,8975 | 60 |

Figure 7A

| Downstream gene | Characteristics of promoter | Score | SEQ ID NO: |
|---|---|---|---|
| TM0373<br>dnaK protein<br>Operon: 2 genes | TTTTACAAATTCTCATACCTTGACATccc<br>attctgtgcctcacTATAAT | 0,94 | 61 |
| TM0505<br>groES protein<br>Operon: 3 genes | TGTTTTTCTTAATCAATCCTTGAAGagg<br>ctcgtaaaaagtagTATATT | 0,8525 | 62 |
| TM0657<br>rubrerythrin<br>Operon: 3 genes | TAATGTAACTATTCAAATCATTACAGTtt<br>ataattatgtggTAAAAT | 0,8125 | 63 |
| TM0682<br>hypothetical protein<br>Operon: 3 genes | GAATACTCTGTCAGAAATCGTGATCAT<br>cttttcacctcgtgtagTATAAT | 0,915 | 64 |
| TM0759<br>putative acyltransferase<br>Operon: 2 genes | ATTTTAATGTTAAATTTTAACGACATggg<br>tggtaaaatctttccaGATAAT | 0,8225 | 65 |
| tRNA-Leu-5<br>Operon: 11 genes | CAATTTTTTCATATCATTCTTATAGTggc<br>actgctgaactcTATATT | 0,8225 | 66 |
| TM0946<br>hypothetical protein | AAAAGAATTGCCAGAAATCATGTTATct<br>ccccctccagtTATAAA | 0,8 | 67 |
| TM1016<br>hypothetical protein | AAAAATTTCATGAAAAATCTTGAATtctgt<br>gaccaaaagggTTTAAT | 0,9175 | 68 |
| TM1058<br>glutamate synthase-related protein | TACAATATTTTGACTGCTCTTGTAGTcct<br>ctctgtttgtttTATAAT | 0,8475 | 69 |
| TM1167<br>hypothetical protein | GAAAAGTTACAGAAAAACCTTGTTATct<br>gaaggtgaaaaatggTAAAAT | 0,865 | 70 |

**Figure 7B**

| Downstream gene | Characteristics of promoter | Score | SEQ ID NO: |
|---|---|---|---|
| tRNA-Asn-1 | TCATTCATTTTACCATCCACTTGAAATtc aggaaggtatgtagTACAAT | 0,8675 | 71 |
| TM1205 hypothetical protein Operon: 13 genes | TTTTTTATCTCTACTAAGGTTGACATtatt gattcagaagagTAAAAT | 0,88 | 72 |
| TM1272 glutamyl tRNA-Glnamidotransferase, subunit A Operon: 3 genes | AAATATAGATTTTCACATTTGCATtataca ccttgacatggTAGAAT | 0,875 | 73 |
| TM1318 Operon: 2 genes | AGAAACAATTTTGGAATCCATGGACATt attacctttaatgGATAAT | 0,8325 | 74 |
| TM1323 hypothetical protein | AAGAATTCTCTTACAATCCTGGAATgttt ccctcacagagaaGATAAT | 0,86 | 75 |
| TM1333 hypothetical protein | AAGAATTCTCTTACAATCCTGGAATgttt ccctcacagagaaGATAAT | 0,86 | 76 |
| tRNA-Leu-2 | AGAAAAATTTCCGATGAACTTGAAAAg ggtgaaaacctgtgcTATTAT | 0,855 | 77 |
| TM1429 glycerol uptake facilitator protein Operon: 3 genes | ATTGTGATTTTTGTAACTATTGACATaa aacaaaaggtttgtTATAAT | 0,9175 | 78 |
| TM1570 hypothetical protein Operon: 3 genes | ATCTTTTCTTAGCGAAGACTGGACGAA atggacaaattggcTATAAT | 0,8175 | 79 |
| tRNA-Ala-1 Operon: 3 genes | AAAAATAAAAAGTCCTTGATTGACCAT atttcgtactcatgcTATAAT | 0,8725 | 80 |

Figure 7C

| Downstream gene | Characteristics of promoter | Score | SEQ ID NO: |
|---|---|---|---|
| TM1667<br>xylose isomerase<br>Operon: 2 genes | AAGTATATCCTAAAAAATTTGAAATgata<br>ccccaagattttaTATAAT | 0,905 | 81 |
| TM1752<br>endoglucanase | TATGATACTCTGAGAAACCTGGAATaa<br>agatcttttagaagcTTTAAT | 0,8525 | 82 |
| TM1780<br>argininosuccinate synthase<br>Operon: 6 genes | AAAATAACAGTGAAAAAACTTCATATaa<br>atcatttcaaataatccTATAAT<--- | 0,875 | 83 |
| TM0032 (complem.)<br>XylR-related trans-criptional regulator | AATATTAGAATTTGAACAATTCGAAATa<br>attcctgttattcactCATAAT | 0,86 | 84 |
| TM0150 (complem.)<br>ribosomal protein L32<br>Operon: 5 genes | AAAAATGTAAAAGAAGAACTTGAATcttt<br>gaaaaacatcaTATACT | 0,855 | 85 |
| TM0153 (complem.)<br>hypothetical protein | AATTCAAATTACGTATAATTTGAATtcac<br>acataattattaCATAAT | 0,85 | 86 |
| TM0237 (complem.)<br>UDP-*N*-acetyl-muramoylalanyl-D-glutamate--2<br>Operon: 6 genes | AAATCATTTTTCTTACCTCTGGAAAAgct<br>ttaaataaagtgtTAAAAT | 0,85 | 87 |
| TM0289 (complem.)<br>6-phosphofructo-kinase, pyrophos-phate-dependent | CTTTTCATTTCAAAAAATTTAACACTttcg<br>cagaaaaattggTAGAAT | 0,8425 | 88 |
| TM0296 (complem.)<br>fructokinase | AAAATAACAGTTCAACATATTAACACac<br>ttcgccttgaagTATAAT | 0,8175 | 89 |
| TM0389 (complem.)<br>ABC transporter, ATP-binding protein<br>Operon: 2 genes | AACAATTCTTTAGATGTTCTGGATACatt<br>ttgattagtttcAATAAT | 0,8225 | 90 |

**Figure 7D**

| Downstream gene | Characteristics of promoter | Score | SEQ ID NO: |
|---|---|---|---|
| TM0401 (complem.) thymidine kinase | AAATATGTTTGTTGACTTTTTAAGATtaa ttctctatcaaTATGAT | 0,8 | 91 |
| TM0423 (complem.) glycerol dehydrogenase | ATAAAAATTTTTCTATATCGTGAAAAattt aacaattaaggttGATAAT | 0,89 | 92 |
| TM0477 (complem.) outer membrane protein alpha | AAAAAAACTTTAGAAAATCTTGAATttcct ttggacgggatggTATAAT | 0,9425 | 93 |
| TM0625 (complem.) hypothetical protein | TGATATTCGTTCTGAATTTTTACATttcat ccaaattattttggtTATAGT | 0,805 | 94 |
| TM0656 (complem.) hypothetical protein Operon: 2 genes | AACTTAAGTAACACAAACCTTGACAAcg aaagggggggtgggTATAAT | 0,8925 | 95 |
| TM0755 (complem.) hypothetical protein Operon: 2 genes | AGAAATTCTTTGAAAACTCTAGAATtcaa acgtcgcttttccagTATACT | 0,85 | 96 |
| TM0815 (complem.) hypothetical protein | TCAATTATTTTACCAAAGGTTCACCATa cgaactatttgttgTAGAAT | 0,86 | 97 |
| TM0960 (complem.) ribokinase Operon: 2 genes | TTCTATATTATGAAAATTTCATGGATATt atccaaaaaattcattTATCAT | 0,8675 | 98 |
| TM0971 (complem.) hypothetical protein | AAATATAAATCTGAATTAATTCACATtta gcaaatcatcattTATAAT | 0,895 | 99 |
| TM0999 (complem.) hypothetical protein | AAACTGCTTTTAAAAAAGATTACATtccg cagtaaacagaattTATTAT | 0,8575 | 100 |
| TM1015 (complem.) glutamate dehydrogenase | CGATAATTTTTGCAATTTCTATACATctc acatcacctccggctaTATATT | 0,855 | 101 |

**Figure 7E**

| Downstream gene | Characteristics of promoter | Score | SEQ ID NO: |
|---|---|---|---|
| TM1067 (complem.) oligopeptide ABC transporter | ATTATTTTATACTGAAACCTTGACCTtgtt gtatgtttgttgaTATTAT | 0,92 | 102 |
| TM1271 (complem.) type IV pilin-related protein | TGATATTTCAACATTAATCTTGACATtct accatgtcaaggtgTATAAT | 0,9525 | 103 |
| TM1286 (complem.) 5-methyltetrahydro-pteroyltriglutamate ... | AAAATGTTTATGCAAATTTCTGTTAACC ATgttacacacaacatgtggTATC | 0,8725 | 104 |
| TM1314 (complem.) conserved hypothetical protein | TCATATTTTGTGTAATTTCCTAACGTtac acagcagtgtgaTAAAAT | 0,83 | 105 |
| tRNA-Leu-1 (complem.) | AAGTTTTGATTTTTGTAGGTTGAAATaat ctttctgacgatgtggTATAAT | 0,86 | 106 |
| TM1412 (complem.) hypothetical protein | ATATGGAAGTTCAAAAATCTTGCTTtcag agtgtgtttgtggTATAAA | 0,865 | 107 |
| TM1419 (complem.) myo-inositol-1-phosphate synthase Operon: 4 genes | AGAAAACTATTGGTAAAACTTGAAATat atgactgtaaaaacgtgaTATAAT | 0,87 | 108 |
| TM1439 (complem.) hypothetical protein Operon: 3 genes | TAGTATTCTACCCTAAATCTTTCATtctgg attcgataatTGTAAT | 0,835 | 109 |
| tRNA-Ser-1 (complem.) | AAAAAGAAGGAAGAAAAAACTTGAACA Aggaaacgattgagtg TATAAT | 0,86 | 110 |
| TM1786 (complem.) hypothetical protein | GTATTATTCATTCTAAAACTTGAAACTg accaaataaagtatTAGAAT | 0,855 | 111 |
| TM1839 (complem.) maltose ABC transporter | TAAAATCCTTAGTGAAATTTGTGAATtttc tgacggtaaactcttTGTAAT | 0,8375 | 112 |
| TM1850 (complem.) hypothetical protein | AAACGATTCTTCTAAAATCTTGATTTgta tcactgttatgtTATAAA | 0,855 | 113 |

**Figure 7F**

**LOCUS: AE000512 1860725 bp DNA circular BCT**
**DEFINITION: *T. maritima* complete genome.**

| No. | Gene number | Gene product | Position of UP | Total score | sUP | s35 | * | *s10* |
|---|---|---|---|---|---|---|---|---|
| 1 | TM1271 | type IV pilin-related protein | 1297213 | 0,9525 | 13 | 8,5 | 17 | 6 |
| 2 | TM0477 | outer membrane protein alpha | 505399 | 0,9425 | 15,5 | 7 | 18 | 6 |
| 3 | TM0373 | dnaK protein | 392176 | 0,94 | 13 | 8 | 17 | 6 |
| 4 | TM1067 | oligopeptide ABC transporter, periplasmi | 1083542 | 0,92 | 15 | 7 | 17 | 5 |
| 5 | TM1016 | hypothetical protein | 1034631 | 0,9175 | 15,5 | 7 | 16 | 5 |
| 6 | TM1429 | glycerol uptake facilitator protein | 1445230 | 0,9175 | 13 | 7,5 | 16 | 6 |
| 7 | TM0682 | hypothetical protein | 708661 | 0,915 | 13 | 7 | 17 | 6 |
| 8 | TM1667 | xylose isomerase | 1653376 | 0,905 | 14 | 6 | 17 | 6 |
| 9 | TM0339 | hypothetical protein | 358527 | 0,8975 | 13,5 | 7 | 17 | 5 |
| 10 | TM1272 | glutamyl tRNA-Gln amidotransferase, subunit A | 1297150 | 0,8975 | 12,5 | 8 | 16 | 5 |
| 11 | TM0971 | hypothetical protein | 986384 | 0,895 | 14 | 6 | 16 | 6 |
| 12 | TM1235 | conserved hypothetical protein | 1263666 | 0,895 | 12,5 | 7,5 | 17 | 5 |
| 13 | TM0656 | conserved hypothetical protein | 688091 | 0,8925 | 13,5 | 7 | 15 | 6 |
| 14 | TM0423 | glycerol dehydrogenase | 441938 | 0,89 | 15,5 | 5,5 | 17 | 5 |
| 15 | TM0702 | chemotaxis sensor histidine kinase CheA | 725066 | 0,885 | 12,5 | 6,5 | 18 | 6 |
| 16 | tRNA-Thr1 | K+ channel, beta subunit | 348341 | 0,8825 | 12,5 | 7 | 17 | 5 |
| 17 | TM0280 | hypothetical protein | 294628 | 0,88 | 14 | 6 | 17 | 5 |
| 18 | TM1205 | conserved hypothetical protein | 1230514 | 0,88 | 13 | 7 | 16 | 5 |
| 19 | TM0122 | ferric uptake regulation protein | 130041 | 0,8775 | 12 | 7,5 | 16 | 5 |
| 20 | tRNA-Val2 | hypothetical protein | 1431074 | 0,8775 | 12 | 7,5 | 16 | 5 |
| 21 | TM0277 | DNA-binding protein, HU | 291365 | 0,875 | 14,5 | 5,5 | 17 | 5 |
| 22 | TM1780 | argininosuccinate synthase | 1755953 | 0,875 | 14 | 6 | 19 | 6 |
| 23 | TM0432 | sugar ABC transporter, periplasmic sugar-binding | 453733 | 0,875 | 12 | 7 | 17 | 5 |
| 24 | TM0312 | conserved hypothetical protein | 336113 | 0,8725 | 12,5 | 6 | 16 | 6 |
| 25 | TM1275 | hypothetical protein | 1300981 | 0,8725 | 12,5 | 6 | 16 | 6 |

**Figure 8A**

| No. | Gene number | Gene product | Position of UP | Total score | sUP | s35 | * | *s10* |
|---|---|---|---|---|---|---|---|---|
| 26 | tRNA-Ala1 | ribosomal protein L19 | 1580223 | 0,8725 | 13 | 6,5 | 15 | 6 |
| 27 | TM0372 | cation efflux system protein, putative | 392110 | 0,8725 | 13,5 | 5 | 17 | 6 |
| 28 | TM0486 | conserved hypothetical protein | 518150 | 0,8725 | 12,5 | 7 | 18 | 5 |
| 29 | TM0994 | hypothetical protein | 1016986 | 0,8725 | 12,5 | 6 | 16 | 6 |
| 30 | TM1286 | 5-methyltetrahydropteroyltriglutamate- | 1315356 | 0,8725 | 15 | 5,5 | 18 | 5 |
| 31 | TM0061 | endo-1,4-beta-xylanase A | 59984 | 0,87 | 12,5 | 5,5 | 17 | 6 |
| 32 | TM1237 | conserved hypothetical protein | 1263564 | 0,87 | 15 | 5 | 17 | 5 |
| 33 | TM0050 | iron(II) transport protein A | 47081 | 0,8675 | 14 | 5,5 | 17 | 5 |
| 34 | TM0300 | oligopeptide ABC transporter, periplasmi | 318096 | 0,8675 | 14 | 5,5 | 17 | 5 |
| 35 | tRNA-Asn1 | hypothetical protein | 1204081 | 0,8675 | 13 | 6,5 | 16 | 5 |
| 36 | TM0960 | ribokinase | 978310 | 0,8675 | 14 | 5,5 | 17 | 5 |
| 37 | TM1167 | hypothetical protein | 1181754 | 0,865 | 13 | 6 | 17 | 5 |
| 38 | TM1412 | hypothetical protein | 1428313 | 0,865 | 13 | 6 | 17 | 5 |
| 39 | tRNA-Ser1 | aminotransferase, class V | 1755746 | 0,865 | 12,5 | 6,5 | 15 | 6 |
| 40 | TM0384 | anaerobic ribonucleoside-triphosphat | 402610 | 0,8625 | 14,5 | 5 | 17 | 5 |
| 41 | TM0972 | conserved hypothetical protein, GGDEF domain | 986454 | 0,86 | 12,5 | 5,5 | 18 | 6 |
| 42 | TM1323 | hypothetical protein | 1342364 | 0,86 | 13,5 | 5,5 | 17 | 5 |
| 43 | TM1333 | hypothetical protein | 1352392 | 0,86 | 13,5 | 5,5 | 17 | 5 |
| 44 | TM0032 | transcriptional regulator, XylR-related | 32618 | 0,86 | 13,5 | 5,5 | 17 | 5 |
| 45 | TM0815 | conserved hypothetical protein | 840383 | 0,86 | 15,5 | 5,5 | 15 | 5 |
| 46 | tRNA-leu1 | conserved hypothetical protein | 1385785 | 0,86 | 13 | 6 | 19 | 6 |
| 47 | TM1368 | ABC transporter, ATP-binding protein | 1385759 | 0,8575 | 12,5 | 5 | 17 | 6 |
| 48 | TM0999 | hypothetical protein | 1019310 | 0,8575 | 14 | 5,5 | 18 | 5 |
| 49 | TM1223 | oligopeptide ABC transporter, periplasmi | 1247829 | 0,8575 | 12,5 | 6 | 17 | 5 |
| 50 | TM1292 | iron-sulfur cluster-binding protein, putative | 1319127 | 0,8575 | 12,5 | 5 | 17 | 6 |

**Figure 8B**

European Patent Office

# EUROPEAN SEARCH REPORT

Application Number
EP 03 29 0203

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE EMBL [Online] 4 June 1999 (1999-06-04), NELSON K.E. ET AL.: "Thermotoga maritima section 94 of 136 of the complete sequence genome" XP002246879 accession no. EBI Database accession no. AE001782; AE000512 * abstract * -& NELSON K.E. ET AL.: "Evidence for lateral gene transfer between Archaea and bacteria from genome sequence of Thermotoga maritima" NATURE, vol. 399, no. 6734, 27 May 1999 (1999-05-27), pages 323-329, XP002905952 * page 328, first paragraph of Methods * ----- | 10-27 | C12Q1/68<br>G06F19/00 |
| X,D | ESTREM ST ET AL: "Identification of an UP element consensus sequence for bacterial promoters" PROCEEDINGS OF THE NATIONAL ACADEMY OF SCIENCES OF USA, NATIONAL ACADEMY OF SCIENCE. WASHINGTON, US, vol. 95, August 1998 (1998-08), pages 9761-9766, XP002182863 ISSN: 0027-8424 * abstract; figures 1,2,6 * ----- | 1-9 | TECHNICAL FIELDS SEARCHED (Int.Cl.7)<br>C12Q<br>G06F |
| A | ROSS WILMA ET AL: "A third recognition element in bacterial promoters: DNA binding by the alpha subunit of RNA polymerase." SCIENCE (WASHINGTON D C), vol. 262, no. 5138, 1993, pages 1407-1413, XP001153419 ISSN: 0036-8075 * abstract * ----- | 1-27 | |

-/--

~~The present search report has been drawn up for all claims~~

| Place of search | Date of completion of the search | Examiner |
|---|---|---|
| Munich | 14 July 2003 | Barz, W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 03 29 0203

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | ESTREM SHAWN T ET AL: "Bacterial promoter architecture: Subsite structure of UP elements and interactions with the carboxy-terminal domain of the RNA polymerase alpha subunit." GENES & DEVELOPMENT, vol. 13, no. 16, 15 August 1999 (1999-08-15), pages 2134-2147, XP002246971 ISSN: 0890-9369 * abstract * ----- | 1-27 | |
| A | US 6 194 159 B1 (LAROSSA ROBERT ALAN ET AL) 27 February 2001 (2001-02-27) * abstract; claim 13 * ----- | 1-27 | |
| A | WERNER THOMAS: "Target gene identification from expression array data by promoter analysis" BIOMOLECULAR ENGINEERING, ELSEVIER, NEW YORK, NY, US, vol. 17, no. 3, March 2001 (2001-03), pages 87-94, XP002196202 ISSN: 1389-0344 * abstract; figures 3,4 * ----- | 1-9 | TECHNICAL FIELDS SEARCHED (Int.Cl.7) |
| A | WERNER T: "Cluster analysis and promoter modeling as bioinformatics tools for the identification of target genes from expression array data" PHARMACOGENOMICS, ASHLEY PUBLICATIONS, GB, vol. 2, no. 1, February 2001 (2001-02), pages 25-36, XP001007617 ISSN: 1462-2416 * abstract * ----- -/-- | 1-9 | |

~~The present search report has been drawn up for all claims~~

| Place of search | Date of completion of the search | Examiner |
|---|---|---|
| Munich | 14 July 2003 | Barz, W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 03 29 0203

| Category | Citation of document with indication, where appropriate, of relevant passages | Relevant to claim | CLASSIFICATION OF THE APPLICATION (Int.Cl.7) |
|---|---|---|---|
| A | VANET ANNE ET AL: "Inferring regulatory elements from a whole genome. An analysis of Helicobacter pylori sigma80 family of promoter signals." JOURNAL OF MOLECULAR BIOLOGY, vol. 297, no. 2, 24 March 2000 (2000-03-24), pages 335-353, XP002246876 ISSN: 0022-2836 * abstract; figures 1-5 * | 1-9 | |
| A | ROSS WILMA ET AL: "Escherichia coli promoters with UP elements of different strengths: Modular structure of bacterial promoters." JOURNAL OF BACTERIOLOGY, vol. 180, no. 20, October 1998 (1998-10), pages 5375-5383, XP002246877 ISSN: 0021-9193 * abstract; figures 1-5 * | 1-9 | TECHNICAL FIELDS SEARCHED (Int.Cl.7) |
| A | CROWLEY EVELYN M ET AL: "A statistical model for locating regulatory regions in genomic DNA." JOURNAL OF MOLECULAR BIOLOGY, vol. 268, no. 1, 1997, pages 8-14, XP002246878 ISSN: 0022-2836 * abstract; figures 1-4 * | 1-9 | |
| | -/-- | | |

~~The present search report has been drawn up for all claims~~

| Place of search | Date of completion of the search | Examiner |
|---|---|---|
| Munich | 14 July 2003 | Barz, W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 03 29 0203

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | BOWRIN V ET AL: "The alpha subunit of RNA polymerase specifically inhibits expression of the porin genes ompF and ompC in vivo and in vitro in Escherichia coli" FEMS MICROBIOLOGY LETTERS, AMSTERDAM, NL, vol. 115, no. 1, 1994, pages 1-6, XP001022294 ISSN: 0378-1097 * the whole document * ----- | 17-25 | |
| | | | TECHNICAL FIELDS SEARCHED (Int.Cl.7) |

~~The present search report has been drawn up for all claims~~

| Place of search | Date of completion of the search | Examiner |
|---|---|---|
| Munich | 14 July 2003 | Barz, W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number

EP 03 29 0203

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-9 completely; 10-27 partially

European Patent Office

**LACK OF UNITY OF INVENTION**
**SHEET B**

Application Number
EP 03 29 0203

The Search Division considers that the present European patent application does not comply with the requirements of unity of invention and relates to several inventions or groups of inventions, namely:

Invention 1: Claims 1-9 (completely), claims 10-27 (partially)

Method for identifying nucleic acids carrying a putative strong bacterial promoter, computer program for performing said method, storage medium for said computer program, method for isolating nucleic acids having strong bacterial promoter activity, isolated nucleic acid having SEQ ID NO:4 (or 70% identity thereto or hybridizing thereto under stringent conditions), expression vector or DNA template comprising said isolated nucleic acid, use of said template for RNA or polypeptide synthesis or for screening antibacterial agents.

---

Invention 2: Claims 10-27 (partially)

Isolated nucleic acid having SEQ ID NO:5 (or 70% identity thereto or hybridizing thereto under stringent conditions), expression vector or DNA template comprising said isolated nucleic acid, use of said template for RNA or polypeptide synthesis or for screening antibacterial agents.

---

Inventions 3-13: Claims 10-27 (partially)

Same as invention 2, but wherein the isolated nucleic acid has SEQ ID NO:s 6-16, respectively (or 70% identity thereto or hybridizing thereto under stringent conditions).

---

Invention 14: Claims 10-27 (partially)

Same as invention 2, but wherein the isolated nucleic acid comprises SEQ ID NO:s 21.

---

## ANNEX TO THE EUROPEAN SEARCH REPORT ON EUROPEAN PATENT APPLICATION NO. EP 03 29 0203

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2003

| Patent document cited in search report | Publication date | Patent family member(s) | Publication date |
|---|---|---|---|
| US 6194159 B1 | 27-02-2001 | US 6025131 A | 15-02-2000 |

EPO FORM P0459

For more details about this annex : see Official Journal of the European Patent Office, No. 12/82

SEQUENCE LISTING

<110> UNIVERSITE DE NANTES

<120> METHOD FOR THE IDENTIFICATION AND ISOLATION OF STRONG BACTERIAL PROMO TERS

<130> B5588-EP

<160> 113

<170> PatentIn version 3.1

<210> 1
<211> 17
<212> DNA
<213> Artificial Sequence

<220>
<223> Promoter

<220>
<221> misc_feature
<222> (1)..(17)
<223> \N is unknown

<400> 1
aaawwtwttt tnnnaaa 17

<210> 2
<211> 9
<212> DNA
<213> Artificial Sequence

<220>
<223> Promoter

<400> 2
tcttgacat 9

<210> 3
<211> 6
<212> DNA
<213> Artificial Sequence

<220>
<223> Promoter

<400> 3
tataat 6

<210> 4
<211> 115
<212> DNA
<213> Thermotoga maritima

<400> 4
cggtttgtct ttgagacgaa tttaacagtt ttcttctgtt cttagcgggt gatatttcaa 60

cattaaaatc ttgacattct accatgtcaa ggtgtataat gcaaaatgtg aaaat 115

<210> 5
<211> 105
<212> DNA
<213> Thermotoga maritima

<400> 5
atattcgatt tccctcatat ttaggtgcat gtatgttttt acaaattctc atacgacccc 60

ttgacatccc attctgtgcc tcactataat ttttcatgga tgaga 105

<210> 6
<211> 142
<212> DNA
<213> Thermotoga maritima

<400> 6
ggggttgtaa gcaaaaggaa aactaatata atcaataata atcaaccata tttatctctt 60

atagttcgat attaggatta ttttatactg aaagcccttg accttgttgt atgtttgttg 120

atattatatt gataacttca ag 142

<210> 7
<211> 131
<212> DNA
<213> Thermotoga maritima

<400> 7
gaaagaagac gtggaaagat ttaaaattta acagaaaata acaacttcca cataagatga 60

aactgcattg tgatttttgt aactatattg acataaaaca aaaggtttgt tataattcac 120

atcgaggcat a 131

<210> 8
<211> 123
<212> DNA
<213> Thermotoga maritima

<400> 8
cctctctgag ctcttctatt ctttttgtga tctccattga aaacacctcc cagattcaag 60

tatatcctaa aaaaatattt gaaatgatac cccaagattt tatataattg attgatagaa 120

aaa 123

<210> 9
<211> 111
<212> DNA
<213> Thermotoga maritima

<400> 9
ctcataaaat ccacctcccg ggttaaaaat tgttaaatat agattttcac attttgcatt 60

atacaccttg acatggtaga atgtcaagat tttaatgttg aaatatcacc c 111

<210> 10

<211> 119
<212> DNA
<213> Thermotoga maritima

<400> 10
gatattcata aacacgaaaa taatatgtgg atttcatcct ttacaaaact gaaaataaca 60

gtgaaaaaac acttcatata aatcatttca aataatccta taattatgct atcaagaac 119

<210> 11
<211> 110
<212> DNA
<213> Thermotoga maritima

<400> 11
gagagttgga aagaggaagt tctacagaat atcaggtgga gagacaaaaa aactttagaa 60

aactcttgaa tttcctttgg acgggatggt ataatcacca caggatttaa 110

<210> 12
<211> 111
<212> DNA
<213> Thermotoga maritima

<400> 12
gccaggataa agaccattct cagagagagg gagttaggca taaggaggtg aaaatatgcc 60

caggaaacgt ttgactggaa tagttgtgag cgataaaatg gacaagacag t 111

<210> 13
<211> 119
<212> DNA
<213> Thermotoga maritima

<400> 13
catctttgca cttttcgttt tcgccgtggg ggtatggaaa tatttcagaa tgaaaaagaa 60

ggaagaaaaa tgaaaacttg aacaaggaaa cgattgagtg tataatattt ttctggtgt 119

<210> 14
<211> 111
<212> DNA
<213> Thermotoga maritima

<400> 14
taccaaggta cgtggtgaat aaagaagtga tccgaatttt gaaagaaaag ggttatcagg 60

aaatatcttg aatagaaaag gttcgtgtgt taaaataacc acacatacgg g 111

<210> 15
<211> 116
<212> DNA
<213> Thermotoga maritima

<400> 15
gctccttgga aagagcatcg ggaataaaat cagttgtaac tcaaagaaaa tattagaatt 60

tgaactataa ttcgaaataa ttcctgttat tcactcataa tcataaaaaa tgagta 116

<210> 16
<211> 103
<212> DNA
<213> Thermotoga maritima

<400> 16
ccatatcgtt acctattgta tcattttgga aacaaaaata aaaatttcat gaaaaatttc 60

ttgaattctg tgaccaaaag ggtttaatat atagccatac ggg 103

<210> 17
<211> 41
<212> DNA
<213> B. Stearothermophilus

<400> 17
ccatggctat agaatttgtg ataccaaaaa aattgaaggt g 41

<210> 18
<211> 27
<212> DNA
<213> B. Stearothermophilus

<400> 18
gtcgacttcc cccttcctga gctcaag 27

<210> 19
<211> 23
<212> DNA
<213> B. Stearothermophilus

<400> 19
ggagggggaa catatgatga agc 23

<210> 20
<211> 20
<212> DNA
<213> B. Stearothermophilus

<400> 20
ggaccaccgc gctactgccg 20

<210> 21
<211> 53
<212> DNA
<213> Artificial Sequence

<220>
<223> Promoter

<220>
<221> misc_feature
<222> (1)..(53)
<223> \N is unknown

<400> 21
gnaaaaatwt nttnaaaaaa mncttgaman nnnnnnnnnn nnnnnnntat aat 53

<210> 22
<211> 8
<212> DNA
<213> E. coli

<400> 22
gctggtgg 8

<210> 23
<211> 18
<212> DNA
<213> Artificial Sequence

<220>
<223> Primer

<400> 23
gcgccgacat cataacgg 18

<210> 24
<211> 38
<212> DNA
<213> Artificial Sequence

<220>
<223> Primer

<400> 24
catatgttcc ccctcctcac aattccacac attatacc 38

<210> 25
<211> 20
<212> DNA
<213> Artificial Sequence

<220>
<223> Primer

<400> 25
gctccttgga aagagcatcg 20

<210> 26
<211> 36
<212> DNA
<213> Artificial Sequence

<220>
<223> Primer

<400> 26
catatgttcc ccctcctact catttttat tatgag 36

<210> 27
<211> 25
<212> DNA
<213> Artificial Sequence

<220>
<223> Primer

<400> 27
atattcgatt tccctcatat ttagg 25

<210> 28
<211> 38
<212> DNA
<213> Artificial Sequence

<220>
<223> Primer

<400> 28
catatgttcc ccctcctctc atccatgaaa aattatag 38

<210> 29
<211> 19
<212> DNA
<213> Artificial Sequence

<220>
<223> Primer

<400> 29
gagagttgga aagaggaag 19

<210> 30
<211> 36
<212> DNA
<213> Artificial Sequence

<220>
<223> Primer

<400> 30
catatgttcc ccctccttaa atcctgtggt gattat 36

<210> 31
<211> 19
<212> DNA
<213> Artificial Sequence

<220>
<223> Primer

<400> 31
ccatatcgtt tacctattg 19

<210> 32
<211> 46
<212> DNA
<213> Artificial Sequence

<220>
<223> Primer

<400> 32

catatgttcc ccctcccccg tatggctata tattaaaccc ttttgg 46

<210> 33
<211> 18
<212> DNA
<213> Artificial Sequence

<220>
<223> Primer

<400> 33
ggggttgtaa gcaaaagg 18

<210> 34
<211> 39
<212> DNA
<213> Artificial Sequence

<220>
<223> Primer

<400> 34
catatgttcc ccctcccttg aagttatcaa tataatatc 39

<210> 35
<211> 21
<212> DNA
<213> Artificial Sequence

<220>
<223> Primer

<400> 35
cggtttgtct ttgagacgaa t 21

<210> 36
<211> 38
<212> DNA
<213> Artificial Sequence

<220>
<223> Primer

<400> 36
catatgttcc ccctccattt tcacattttg cattatag 38

<210> 37
<211> 18
<212> DNA
<213> Artificial Sequence

<220>
<223> Primer

<400> 37
cccgctctct ttctcatt 18

<210> 38

<211> 38
<212> DNA
<213> Artificial Sequence

<220>
<223> Primer

<400> 38
catatgttcc ccctccatta aaatcttgac attctacc 38

<210> 39
<211> 18
<212> DNA
<213> Artificial Sequence

<220>
<223> Primer

<400> 39
gaaagaagac gtggaaag 18

<210> 40
<211> 39
<212> DNA
<213> Artificial Sequence

<220>
<223> Primer

<400> 40
catatgttcc ccctcctatg cctcgatgtg aattataac 39

<210> 41
<211> 19
<212> DNA
<213> Artificial Sequence

<220>
<223> Primer

<400> 41
gccaggataa agaccattc 19

<210> 42
<211> 36
<212> DNA
<213> Artificial Sequence

<220>
<223> Primer

<400> 42
catatgttcc ccctccactg tcttgtccat tttatc 36

<210> 43
<211> 18
<212> DNA
<213> Artificial Sequence

<220>
<223> Primer

<400> 43
cctctctgag ctcttcta 18

<210> 44
<211> 33
<212> DNA
<213> Artificial Sequence

<220>
<223> Primer

<400> 44
catatgttcc ccctcctttt tctatcaatc aat 33

<210> 45
<211> 18
<212> DNA
<213> Artificial Sequence

<220>
<223> Primer

<400> 45
gatattcata aacacgaa 18

<210> 46
<211> 39
<212> DNA
<213> Artificial Sequence

<220>
<223> Primer

<400> 46
catatgttcc ccctccgttc ttgatagcat aattatagg 39

<210> 47
<211> 17
<212> DNA
<213> Artificial Sequence

<220>
<223> Primer

<400> 47
catctttgca cttttcg 17

<210> 48
<211> 38
<212> DNA
<213> Artificial Sequence

<220>
<223> Primer

<400> 48

catatgttcc ccctccacac cagaaaaata ttatacac 38

<210> 49
<211> 18
<212> DNA
<213> Artificial Sequence

<220>
<223> Primer

<400> 49
taccaaggta cgtggtga 18

<210> 50
<211> 54
<212> DNA
<213> Artificial Sequence

<220>
<223> Primer

<400> 50
catatgttcc ccctcccccg tatgtgcccg tatgtgtggt tattttaaca cacg 54

<210> 51
<211> 46
<212> DNA
<213> Thermotoga maritima

<400> 51
acaattttta tctgatattt tcacattcac catagtcgat tataac 46

<210> 52
<211> 47
<212> DNA
<213> Thermotoga maritima

<400> 52
atttattgtt taagtattta tgaaatgcat tatgtatctg atacaat 47

<210> 53
<211> 51
<212> DNA
<213> Thermotoga maritima

<400> 53
agaatgtgtg taacaaacca tcgaaatcat aagttattga ctccatgtaa t 51

<210> 54
<211> 49
<212> DNA
<213> Thermotoga maritima

<400> 54
gacaatatat tagaaattat tgaaagcatc catgtgatga tgatacaat 49

<210> 55

<211> 45
<212> DNA
<213> Thermotoga maritima

<400> 55
accttgattt taaattatcc tgcatataat taatgtgaac ataat 45

<210> 56
<211> 47
<212> DNA
<213> Thermotoga maritima

<400> 56
acaatgtttg aatgatactt gaaatcagcg actgtgtgta gtacaat 47

<210> 57
<211> 50
<212> DNA
<213> Thermotoga maritima

<400> 57
agatttatgt taactaaact ataagataat tctttgttga cagatatgat 50

<210> 58
<211> 49
<212> DNA
<213> Thermotoga maritima

<400> 58
caatatttgt ccagaaaact tgatttaaca aaaatggaca atgtagaat 49

<210> 59
<211> 51
<212> DNA
<213> Thermotoga maritima

<400> 59
aaatgtgata tgaaaaatat ggaacgataa gttatcatat ttctttttaa t 51

<210> 60
<211> 49
<212> DNA
<213> Thermotoga maritima genome

<400> 60
agaaaaattt ttttggaact tgacaaaata tttggtaata ttctaaaat 49

<210> 61
<211> 49
<212> DNA
<213> Thermotoga maritima

<400> 61
ttttacaaat tctcatacct tgacatccca ttctgtgcct cactataat 49

<210> 62
<211> 48

<212> DNA
<213> Thermotoga maritima

<400> 62
tgtttttctt aatcaatcct tgaagaggct cgtaaaaagt agtatatt 48

<210> 63
<211> 47
<212> DNA
<213> Thermotoga maritima

<400> 63
taatgtaact attcaaatca ttacagttta taattatgtg gtaaaat 47

<210> 64
<211> 50
<212> DNA
<213> Thermotoga maritima

<400> 64
gaatactctg tcagaaatcg tgatcatctt ttcacctcgt gtagtataat 50

<210> 65
<211> 51
<212> DNA
<213> Thermotoga maritima

<400> 65
attttaatgt taaattttaa cgacatgggt ggtaaaatct ttccagataa t 51

<210> 66
<211> 47
<212> DNA
<213> Thermotoga maritima

<400> 66
caatttttc atatcattct tatagtggca ctgctgaact ctatatt 47

<210> 67
<211> 46
<212> DNA
<213> Thermotoga maritima

<400> 67
aaaagaattg ccagaaatca tgttatctcc cccctccagt tataaa 46

<210> 68
<211> 47
<212> DNA
<213> Thermotoga maritima

<400> 68
aaaaatttca tgaaaaatct tgaattctgt gaccaaaagg gtttaat 47

<210> 69
<211> 47
<212> DNA

<213> Thermotoga maritima

<400> 69
tacaatattt tgactgctct tgtagtcctc tctgtttgtt ttataat 47

<210> 70
<211> 49
<212> DNA
<213> Thermotoga maritima

<400> 70
gaaaagttac agaaaaacct tgttatctga aggtgaaaaa tggtaaaat 49

<210> 71
<211> 49
<212> DNA
<213> Thermotoga maritima

<400> 71
tcattcattt taccatccac ttgaaattca ggaaggtatg tagtacaat 49

<210> 72
<211> 48
<212> DNA
<213> Thermotoga maritima

<400> 72
ttttttatct ctactaaggt tgacattatt gattcagaag agtaaaat 48

<210> 73
<211> 47
<212> DNA
<213> Thermotoga maritima

<400> 73
aaatatagat tttcacattt gcattataca ccttgacatg gtagaat 47

<210> 74
<211> 47
<212> DNA
<213> Thermotoga maritima

<400> 74
agaaacaatt ttggaatcca tggacattat tacctttaat ggataat 47

<210> 75
<211> 48
<212> DNA
<213> Thermotoga maritima

<400> 75
aagaattctc ttacaatcct ggaatgtttc cctcacagag aagataat 48

<210> 76
<211> 48
<212> DNA
<213> Thermotoga maritima

<400> 76
aagaattctc ttacaatcct ggaatgtttc cctcacagag aagataat 48

<210> 77
<211> 48
<212> DNA
<213> Thermotoga maritima

<400> 77
agaaaaattt ccgatgaact tgaaaagggt gaaaacctgt gctattat 48

<210> 78
<211> 48
<212> DNA
<213> Thermotoga maritima

<400> 78
attgtgattt ttgtaactat tgacataaaa caaaaggttt gttataat 48

<210> 79
<211> 47
<212> DNA
<213> Thermotoga maritima

<400> 79
atcttttctt agcgaagact ggacgaaatg gacaaattgg ctataat 47

<210> 80
<211> 48
<212> DNA
<213> Thermotoga maritima

<400> 80
aaaaataaaa agtccttgat tgaccatatt tcgtactcat gctataat 48

<210> 81
<211> 48
<212> DNA
<213> Thermotoga maritima

<400> 81
aagtatatcc taaaaaattt gaaatgatac cccaagattt tatataat 48

<210> 82
<211> 48
<212> DNA
<213> Thermotoga maritima

<400> 82
tatgatactc tgagaaacct ggaataaaga tcttttagaa gctttaat 48

<210> 83
<211> 51
<212> DNA
<213> Thermotoga maritima

<400> 83
aaaataacag tgaaaaaact tcatataaat catttcaaat aatcctataa t 51

<210> 84
<211> 50
<212> DNA
<213> Thermotoga maritima

<400> 84
aatattagaa tttgaacaat tcgaaataat tcctgttatt cactcataat 50

<210> 85
<211> 46
<212> DNA
<213> Thermotoga maritima

<400> 85
aaaaatgtaa aagaagaact tgaatctttg aaaaacatca tatact 46

<210> 86
<211> 47
<212> DNA
<213> Thermotoga maritima

<400> 86
aattcaaatt acgtataatt tgaattcaca cataattatt acataat 47

<210> 87
<211> 48
<212> DNA
<213> Thermotoga maritima

<400> 87
aaatcatttt tcttacctct ggaaaagctt taaataaagt gttaaaat 48

<210> 88
<211> 48
<212> DNA
<213> Thermotoga maritima

<400> 88
cttttcattt caaaaaattt aacactttcg cagaaaaatt ggtagaat 48

<210> 89
<211> 46
<212> DNA
<213> Thermotoga maritima

<400> 89
aaaataacag ttcaacatat taacacactt cgccttgaag tataat 46

<210> 90
<211> 47
<212> DNA
<213> Thermotoga maritima

<400> 90

aacaattctt tagatgttct ggatacattt tgattagttt caataat 47

<210> 91
<211> 46
<212> DNA
<213> Thermotoga maritima

<400> 91
aaatatgttt gttgactttt taagattaat tctctatcaa tatgat 46

<210> 92
<211> 49
<212> DNA
<213> Thermotoga maritima

<400> 92
ataaaaattt ttctatatcg tgaaaaattt aacaattaag gttgataat 49

<210> 93
<211> 49
<212> DNA
<213> Thermotoga maritima

<400> 93
aaaaaaactt tagaaaatct tgaatttcct ttggacggga tggtataat 49

<210> 94
<211> 51
<212> DNA
<213> Thermotoga maritima

<400> 94
tgatattcgt tctgaatttt tacatttcat ccaaattatt ttggttatag t 51

<210> 95
<211> 47
<212> DNA
<213> Thermotoga maritima

<400> 95
aacttaagta acacaaacct tgacaacgaa agggggtgg gtataat 47

<210> 96
<211> 50
<212> DNA
<213> Thermotoga maritima

<400> 96
agaaattctt tgaaaactct agaattcaaa cgtcgctttt ccagtatact 50

<210> 97
<211> 48
<212> DNA
<213> Thermotoga maritima

<400> 97
tcaattattt taccaaaggt tcaccatacg aactatttgt tgtagaat 48

<210> 98
<211> 51
<212> DNA
<213> Thermotoga maritima

<400> 98
ttctatatta tgaaaatttc atggatatta tccaaaaaat tcatttatca t 51

<210> 99
<211> 48
<212> DNA
<213> Thermotoga maritima

<400> 99
aaatataaat ctgaattaat tcacatttag caaatcatca tttataat 48

<210> 100
<211> 49
<212> DNA
<213> Thermotoga maritima

<400> 100
aaactgcttt taaaaaagat tacattccgc agtaaacaga atttattat 49

<210> 101
<211> 51
<212> DNA
<213> Thermotoga maritima

<400> 101
cgataatttt tgcaatttct atacatctca catcacctcc ggctatatat t 51

<210> 102
<211> 49
<212> DNA
<213> Thermotoga maritima

<400> 102
attattttat actgaaacct tgaccttgtt gtatgtttgt tgatattat 49

<210> 103
<211> 49
<212> DNA
<213> Thermotoga maritima

<400> 103
tgatatttca acattaatct tgacattcta ccatgtcaag gtgtataat 49

<210> 104
<211> 52
<212> DNA
<213> Thermotoga maritima

<400> 104
aaaatgttta tgcaaatttc tgttaaccat gttacacaca acatgtggta tc 52

<210> 105
<211> 47
<212> DNA
<213> Thermotoga maritima

<400> 105
tcatattttg tgtaatttcc taacgttaca cagcagtgtg ataaaat 47

<210> 106
<211> 51
<212> DNA
<213> Thermotoga maritima

<400> 106
aagttttgat ttttgtaggt tgaaataatc tttctgacga tgtggtataa t 51

<210> 107
<211> 48
<212> DNA
<213> Thermotoga maritima

<400> 107
atatggaagt tcaaaaatct tgctttcaga gtgtgtttgt ggtataaa 48

<210> 108
<211> 52
<212> DNA
<213> Thermotoga maritima

<400> 108
agaaaactat tggtaaaact tgaaatatat gactgtaaaa acgtgatata at 52

<210> 109
<211> 46
<212> DNA
<213> Thermotoga maritima

<400> 109
tagtattcta ccctaaatct ttcattctgg attcgataat tgtaat 46

<210> 110
<211> 49
<212> DNA
<213> Thermotoga maritima

<400> 110
aaaaagaagg aagaaaaaac ttgaacaagg aaacgattga gtgtataat 49

<210> 111
<211> 48
<212> DNA
<213> Thermotoga maritima

<400> 111
gtattattca ttctaaaact tgaaactgac caaataaagt attagaat 48

```
<210>  112
<211>  51
<212>  DNA
<213>  Thermotoga maritima

<400>  112
taaaatcctt agtgaaattt gtgaattttc tgacggtaaa ctctttgtaa t             51


<210>  113
<211>  47
<212>  DNA
<213>  Thermotoga maritima

<400>  113
aaacgattct tctaaaatct tgatttgtat cactgttatg ttataaa                  47
```